(19) Europäisches Patentamt
European Patent Office
Office européen des brevets

(11) Veröffentlichungsnummer: **0 222 996 B1**

# (12) EUROPÄISCHE PATENTSCHRIFT

(45) Veröffentlichungstag der Patentschrift: **22.05.91**

(51) Int. Cl.[5]: **C07D 311/58**, C07D 335/06, A61K 31/35, A61K 31/38

(21) Anmeldenummer: **86112104.4**

(22) Anmeldetag: **01.09.86**

Die Akte enthält technische Angaben, die nach dem Eingang der Anmeldung eingereicht wurden und die nicht in dieser Patentschrift enthalten sind.

## (54) 3-Amino-dihydro-[1]-benzopyrane und Benzothiopyrane.

(30) Priorität: **03.09.85 US 772068**

(43) Veröffentlichungstag der Anmeldung:
**27.05.87 Patentblatt 87/22**

(45) Bekanntmachung des Hinweises auf die Patenterteilung:
**22.05.91 Patentblatt 91/21**

(84) Benannte Vertragsstaaten:
**AT BE CH DE FR GB IT LI LU NL SE**

(56) Entgegenhaltungen:
**US-A- 3 607 886**
**US-A- 3 629 289**

**Eur. J. Med. Chem.- Chimica Therapeutica 11,251-256,257-262(1976)**

(73) Patentinhaber: **CIBA-GEIGY AG**
**Klybeckstrasse 141**
**CH-4002 Basel(CH)**

(72) Erfinder: **Hutchison, Alan J.**
**35 Lynwood Road**
**Verona New Jersey 07044(US)**

(74) Vertreter: **Zumstein, Fritz, Dr. et al**
**Bräuhausstrasse 4**
**W-8000 München 2(DE)**

**EP 0 222 996 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung sind 3,4-Dihydro-2H-[1]-(benzopyran und benzothiopyran)-3-amine, welche als Modulatoren für die Rezeptoren des zentralen Nervensystems verwendbar sind, insbesondere als Serotoninrezeptor-Agonisten, beispielsweise für die Behandlung von Störungen des zentralen Nervensystems, insbesondere Depressionen und Angstzuständen, Verfahren zur Herstellung dieser Verbindungen und pharmazeutische Zusammensetzungen, welche diese Verbindungen enthalten.

Aus U.S. Patent 3,607,886 und U.S. Patent 3,629,289 sind 2-Methyl-3,4-dihydro-N,N-dimethyl-2H-[1]-benzopyran-3-amin bzw. 2-Methyl-3,4-dihydro-2H-[1]-benzopyran-3-amin bekannt, die psychostimulierende Eigenschaften, insbesondere eine ausgeprägt antidepressive Wirkung, aufweisen. N. Sarda et al., Eur. J. Med. Chem. - Chimica Therapeutica 11, 251-256 und 257-262 (1976) beschreiben die Synthese und Amphetamin-ähnliche pharmakologische Wirkung von gegebenenfalls in 6- oder 7-Stellung substituierten 3,4-Dihydro-2H-[1]-benzopyran-3-aminen.

Die Erfindung betrifft insbesondere neue 3,4-Dihydro-2H-[1]-benzopyran-3-amin-und 3,4-Dihydro-2H-[1]-benzothiopyran-3-amin-Derivate der Formel

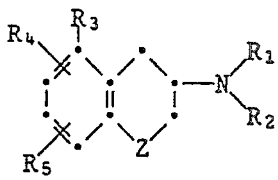

(I A),

worin Z Sauerstoff oder Schwefel, $R_1$ Wasserstoff, $C_1$-$C_4$-Alkyl, Benzyl oder 2-Phenylethyl, $R_2$ Wasserstoff, $C_1$-$C_4$-Alkyl, Benzyl oder 2-Phenylethyl oder $R_1$ und $R_2$ zusammengenommen Alkylen mit 4 bis 6 C-Atomen, $R_3$ Hydroxy, $C_1$-$C_4$-Alkoxy, Phenyl-$C_1$-$C_4$-alkoxy, welches am Phenylring durch 1-3 $C_1$-$C_4$-Alkyl, Halogen oder $C_1$-$C_4$-Alkoxy substituiert sein kann, $C_1$-$C_4$-Alkanoyloxy, Benzoyloxy, $C_1$-$C_4$-Alkoxycarbonyloxy, Carbamoyloxy oder Mono- oder Di-$C_1$-$C_4$-alkylcarbamoyloxy und $R_4$ und $R_5$ unabhängig voneinander Wasserstoff, $C_1$-$C_4$-Alkyl oder Halogen bedeuten, Mono- oder Di-S-Oxide dieser Verbindungen, worin Z Schwefel bedeutet, und pharmazeutisch annehmbare Salze dieser Verbindungen.

Bevorzugter Erfindungsgegenstand sind Verbindungen der Formel I A sowie der Formel I B, welche im folgenden definiert wird, worin 2 Schwefel bedeutet, sowie die Mono- oder Di-S-Oxide und pharmazeutisch annehmbare Salze dieser Verbindungen.

Ein weiterer bevorzugter Erfindungsgegenstand sind Verbindungen der Formel I A sowie der Formel I B, welche im folgenden definiert wird, worin Z Sauerstoff bedeutet, sowie die pharmazeutisch annehmbaren Salze dieser Verbindungen.

Bevorzugt sind Verbindungen der Formel I A, worin Z Sauerstoff oder Schwefel, $R_1$ Wasserstoff oder $C_1$-$C_4$-Alkyl, $R_2$ $C_1$-$C_4$-Alkyl, Benzyl oder 2-Phenylethyl oder $R_1$ und $R_2$ zusammen Butylen oder Pentylen, $R_3$ Hydroxy, $C_1$-$C_4$-Alkoxy, Benzyloxy, Acetoxy, Benzoyloxy, tert.-Butoxycarbonyloxy, Carbamoyloxy oder Mono- oder Di-$C_1$-$C_4$-alkylcarbamoyloxy, $R_4$ Wasserstoff, Halogen oder $C_1$-$C_4$-Alkyl und $R_5$ Wasserstoff bedeuten, und pharmazeutisch annehmbare Salze davon.

Weiterhin sind Verbindungen der Formel I A bevorzugt, worin Z Sauerstoff oder Schwefel, $R_1$ und $R_2$ $C_1$-$C_4$-Alkyl, $R_3$ Hydroxy, $C_1$-$C_4$-Alkoxy, Benzyloxy, Acetoxy, Benzoyloxy, tert.-Butoxycarbonyloxy, Carbamoyloxy oder Mono- oder Di-$C_1$-$C_4$-alkylcarbamoyloxy, $R_4$ Wasserstoff, $C_1$-$C_4$-Alkyl oder Halogen in 7- oder 8-Stellung und $R_5$ Wasserstoff bedeuten, und pharmazeutisch annehmbare Salze davon.

Ein besonders bevorzugter Erfindungsgegenstand betrifft solche Verbindungen der Formel I A, worin Z Sauerstoff bedeutet. Ein weiterer bevorzugter Erfindungsgegenstand betrifft die genannten Verbindungen der Formel I A, worin Z Schwefel bedeutet. Vor allem sind die Verbindungen der Formel I A bevorzugt, worin Z Schwefel darstellt.

Weiterhin sind Verbindungen der Formel

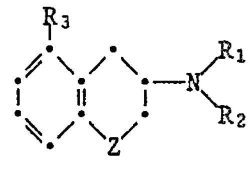

(I B)

bevorzugt, worin Z Sauerstoff oder Schwefel, $R_1$ und $R_2$ $C_1$-$C_4$-Alkyl, Benzyl oder 2-Phenylethyl und $R_3$ Hydroxy, $C_1$-$C_4$-Alkoxy, Benzyloxy, Acetoxy, Benzoyloxy, tert.-Butoxycarbonyloxy, Carbamoyloxy oder Mono- oder Di-$C_1$-$C_4$-alkylcarbamoyloxy bedeuten, und pharmazeutisch annehmbare Salze davon.

Weiterhin sind Verbindungen der Formel I B bevorzugt, worin Z Sauerstoff oder Schwefel, $R_1$ und $R_2$ $C_1$-$C_4$-Alkyl, $R_3$ Hydroxy, $C_1$-$C_4$-Alkoxy, Benzyloxy, Acetoxy, Benzoyloxy, tert.-Butoxycarbonyloxy, Carbamoyloxy oder Mono- oder Di-$C_1$-$C_4$-alkylcarbamoyloxy bedeuten, und pharmazeutisch annehmbare Salze dieser Verbindungen.

Ein besonders bevorzugter Erfindungsgegenstand betrifft solche Verbindungen der Formel I B, worin Z Sauerstoff bedeutet. Ein weiterer bevorzugter Erfindungsgegenstand betrifft die genannten Verbindungen der Formel I B, worin Z Schwefel bedeutet. Vor allem sind die Verbindungen der Formel I B bevorzugt, worin Z Schwefel darstellt.

Die Definitionen in der Beschreibung dieser Erfindung haben z.B. die folgenden Bedeutungen:

$C_1$-$C_4$-Alkyl ist beispielsweise Methyl, Ethyl, n-Propyl oder n-Butyl.

$C_1$-$C_4$-Alkoxy ist z.B. Methoxy oder Ethoxy.

$C_1$-$C_4$-Alkanoyloxy ist z.B. Acetoxy, $C_1$-$C_4$-Alkoxycarbonyloxy ist z.B. tert-Butocarbonyloxy, Di-$C_1$-$C_4$-Alkylcarbamoyloxy ist z.B. Dimethylcarbamoyloxy.

$C_1$-$C_4$-Alkanoyloxy ist vorzugsweise Acetoxy, Pivaloyloxy oder Propionyloxy.

$C_1$-$C_4$-Alkoxycarbonyloxy ist vorzugsweise Methoxycarbonyloxy, Ethoxycarbonyloxy, Propoxycarbonyloxy oder tert-Butoxycarbonyloxy.

Mono- oder Di-$C_1$-$C_4$-alkylcarbamoyloxy ist vorzugsweise Mono- oder Di-N-(Methyl, Ethyl, n-Propyl)-carbamoyloxy.

Halogen ist vorzugsweise Fluor oder Chlor, kann aber auch Brom oder Jod sein.

Die Verbindungen der Formel I A und I B können in Form von Stereoisomeren, z.B. optisch reinen Antipoden, vorliegen, welche ebenfalls Gegenstand der vorliegenden Erfindung sind.

Pharmazeutisch annehmbare Salze sind therapeutisch verwendbare Säureadditionssalze, vorzugsweise Salze von anorganischen oder organischen Säuren, z.B. starken Mineralsäuren, z.B. Halogenwasserstoffsäuren, z.B. Chlorwasserstoff oder Bromwasserstoff, Schwefel-, Phosphor- oder Salpetersäure, aliphatischen oder aromatischen Carbonsäuren oder Sulfonsäuren, z.B. Ameisen-, Essig-, Propion-, Bernstein-, Glycol-, Milch-, Apfel-, Wein-, Zitronen-, Malein-, Fumar-, Brenztrauben-, Phenylessig-, Benzoe-, 4-Aminobenzoe-, Anthranil-, 4-Hydroxybenzoe-, Salicyl-, 4-Aminosalicyl-, Glucon-, Pamoa-, Nicotin-, Methansulfon-, Ethansulfon-, Hydroxyethansulfon-, Benzolsulfon-, p-Toluolsulfon-, Naphthalinsulfon-, Sulfanil-, Cyclohexylsulfamin- oder Ascorbinsäure.

Die neuen Verbindungen der Formel I A und I B sind in bekannten pharmakologischen In-vitro- und In-vivo-Modellen wirksam, welche eine Wirkungsbeziehung hinsichtlich Wirksamkeit bei der Behandlung von Störungen des zentralen Nervensystems bei Säugetieren, vor allem beim Menschen herstellen.

Die erfindungsgemässen Verbindungen sind als selektive Serotonin-(S)-Rezeptor-Stimulatoren (Agonisten) in Säugetieren wirksam, insbesondere mit Selektivität auf den S-1 Rezeptor und vor allem den S-1A Rezeptor.

Daher besitzen die Verbindungen der Formel I A und I B wertvolle pharmakologische Eigenschaften in Säugern, insbesondere Serotonin-(S)-Rezeptor stimulierende (agonistische) Eigenschaften, welche für anxiolytische und psychostimulierende Aktivität indiziert ist.

Die genannten Eigenschaften lassen sich in in-vitro und in-vivo Versuchen, vorzugsweise bei Säugetieren, z.B. Ratten, Hunden oder Affen, aber auch an entnommenen isolierten Organen, Gewebsproben oder enzymatischen Aufbereitungen nachweisen. Die Verbindungen können in-vitro in Form von Lösungen, z.B. wässrigen Lösungen, und in-vivo entweder enteral oder parenteral, mit Vorteil oral oder intravenös, z.B. in Gelatinekapseln, als Stärkesrnspensionen oder in wässrigen Lösungen, appliziert werden. Der Dosisbereich in-vitro kann zwischen $10^{-4}$ und $10^{-9}$ molaren Konzentrationen betragen. Der Dosisbereich in-vivo kann zwischen 0,05 und 30 mg/kg/Tag, vorzugsweise zwischen 0,10 und 1 mg/kg/Tag betragen.

Die Serotonin-(S-1A)-Aufnahme, welche für Regulatoren mit Rezeptoreigenschaften bei Serotonin-Agonisten indiziert ist, kann in-vitro wie folgt in einem Bindungsassay nachgewiesen werden:

Man stellt Suspensionen aus Membranmaterial von Rattenhirn her und inkubiert diese mit $^3H$-Serotonin gemäss der Vorschrift von Martin und Sanders-Bush, Naunyn-Schmiederberg Arch. Pharmacol. 321, 165 (1982) und Middlemiss und Fozard Eur. J. Pharmacol., 90, 151 (1983).

In diesem Bindungsassay werden aliquote Teile von 85 ml Zellsuspensionen zu abgepufferten $^3H$-Serotonin-Lösungen mit und ohne Wirkstoff gegeben. Die Konzentration an $^3H$-Serotonin ist 2 nM und man wertet die zu testenden Wirkstoffe über einen weiten Konzentrationsbereich aus. Die $IC_{50}$-Werte (Konzentrationen an Wirkstoff, welche zur Hemmung von 50 % 2 nM $^3H$-Serotonin nötig sind) werden graphisch bestimmt.

So haben beispielsweise die erfindungsgemässen Verbindungen 5-Hydroxy-3,4-dihydro-N,N-dipropyl-2H-[1]-benzothiopyran-3-aminhydrochlorid $IC_{50}$-Werte von ca. 2,6 x $10^{-9}$ M, 5-Ethoxy-3,4-dihydro-N,N-dipropyl-2H-[1]-benzotiopyran-3-aminhydrochlorid von ca. 2,1 x $10^{-8}$ M und 5-Ethoxy-N,N-dipropyl-3,4-dihydro-2H-[1]-benzopyran-3-amin-hydrochlorid von ca. 4,0 x $10^{-8}$.

Die Serotonin-Rezeptor-Eigenschaften können auch in-vivo durch Abnahme der 5-Hydroxytryptophan-Konzentration in der Hirnrinde von Ratten nach Applikation einer Testverbindung gemäss der in J. Med. Chem. 21, 864 (1978) beschriebenen Methodik bestimmt werden.

So haben beispielsweise die erfindungsgemässen Verbindungen 5-Ethoxy-3,4-dihydro-N,N-dipropyl-2H-[1]-benzothiopyran-3-aminhydrochlorid eine Abnahme der 5-Hydroxytryptophan-Konzentration in der Hirnrinde von Ratten um ca. 25 % nach Verabreichung einer Dosis von ca. 2,7 mg/kg p.o. zur Folge.

Die besagten vorteilhaften Eigenschaften belegen die nützliche therapeutische Verwendbarkeit der erfindungsgemässen Verbindungen in Säugern (inkl. Menschen) als Arzneimittel mit Wirkung auf das zentrale Nervensystem. Die erfindungsgemässen Verbindungen können in erster Linie als Serotonin-Agonisten mit psychostimulierender Wirkung zur Behandlung von Depressionen, Wahrnehmungsstörungen (senile Demenz) und Kleinhirnstörungen, als Anxiolytika bei der Behandlung von Angstzuständen und als Appetitzügler verwendet werden.

Bestimmte erfindungsgemässe Verbindungen besitzen stimulierende (agonistische) Eigenschaften von präsynaptischen Dopaminrezeptoren und daher neuroleptische (antipsychotische) Wirkung.

Die Bindungswirkung auf präsynaptische Dopaminrezeptoren lässt sich in-vitro im Dopamin-Bindungs-Assay nach Standardmethoden durch Ersatz des Dopaminagonisten 2-Amino-6,7-dihydroxy-1,2,3,4-tetrahydronaphthalin ($^3$H-ADTN) aus Membranen von Nucleus caudatus von Kälbern bestimmen.

Die in-vivo Wirkung als präsynaptischer Dopaminagonist lässt sich im Gamma-Butyrolacton (GBL)-Modell an der Ratte anhand der von Walters und Roth in Naunyn-Schmiederberg Arch. Pharmacol. 296, 6 (1976) beschriebenen Methode bestimmen. In diesem pharmazeutischen Modell inhibiert der präsynaptische Dopamin-Agonist die GBL-induzierte Anreicherung der Dopamin-Vorstufe DOPA im Gehirn nach vorheriger Behandlung mit 3-Hydroxybenzylhydrazin (NDS-1015), einem DOPA-Decarboxylase-Inhibitor.

Verbindungen mit agonistischer Wirkung auf präsynaptische Dopaminrezeptoren sind bei der Behandlung von psychotischen Störungen, Parkinsonismus und Bewegungsstörungen verwendbar.

Die erfindungsgemässen Verbindungen sind nach folgenden Verfahren herstellbar, wonach man

a) eine Verbindung der Formel

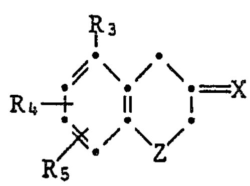

(II),

worin Z und $R_3$-$R_5$ die genannten Bedeutungen haben und X Oxo oder reaktionsfähiges, verestertes Hydroxy zusammen mit Wasserstoff, welches an die Stelle der Doppelbindung tritt, bedeuten, oder ein Mono- oder Di-S-Oxid einer Verbindung der Formel II, worin Z Schwefel bedeutet, mit einer Verbindung der Formel

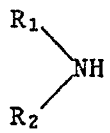

(III),

worin $R_1$ und $R_2$ die genannten Bedeutungen haben, kondensiert oder, falls X Oxo bedeutet, unter reduzierenden Bedingungen kondensiert, oder

b) eine Verbindung der Formel

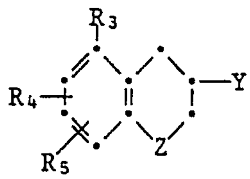

(IV),

worin Z und $R_3$-$R_5$ die genannten Bedeutungen haben und Y $-NH_2$, $-NHR_1$ oder $-NHR_2$ darstellt, mit einem reaktionsfähigen Ester eines Alkohols der Formel

$R_1$-OH oder $R_2$-OH (V),

worin jeweils $R_1$ und $R_2$ die genannten Bedeutungen haben, alkyliert oder mit einem dem Alkohol $R_1$-OH oder $R_2$-OH entsprechenden Aldehyd unter Einhaltung von reduzierenden Bedingungen alkyliert, oder
c) zur Herstellung einer Verbindung I A, worin $R_3$ Hydroxy bedeutet, eine Verbindung der Formel

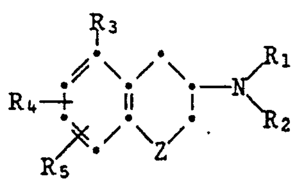

(VI),

worin Z, $R_1$, $R_2$, $R_4$ und $R_5$ die genannten Bedeutungen haben und $R_3$ veräthertes Hydroxy bedeutet, spaltet, oder
d) eine Verbindung der Formel

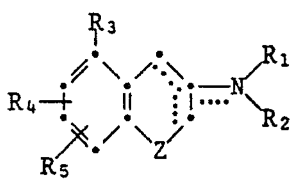

(VII),

worin Z und $R_1$-$R_5$ die genannten Bedeutungen haben und die punktierten Bindungslinien die mögliche Lage einer Doppelbindung in einer der angezeigten Positionen darstellen, reduziert, oder
e) eine Verbindung der Formel

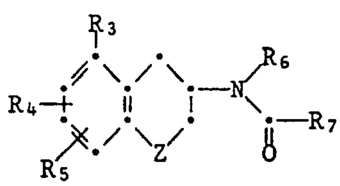

(VIII),

worin $R_6$ die Bedeutung von $R_1$ oder $R_2$ hat, Z und $R_1$-$R_5$ die genannten Bedeutungen haben und $R_7$ Wasserstoff, $C_1$-$C_3$-Alkyl, Phenyl oder Benzyl bedeuten, reduziert,
und, falls erwünscht, reaktionsfähige funktionelle Gruppen bei der Ausführung einer der genannten Verfahrensvarianten schützt und anschliessend die freie Verbindung der Formel I A isoliert und/oder eine erhältliche Verbindung der Formel I A in eine andere Verbindung der Formel I A, worin Z und $R_1$-$R_5$ die genannten Bedeutungen haben, umwandelt und/oder eine erhältliche freie Verbindung in ein Salz oder ein Salz in die freie Verbindung oder in ein anderes Salz umwandelt und/oder ein erhältliches Gemisch von Isomeren in einzelne Isomere auftrennt und/oder ein Racemat in die optischen Antipoden aufspaltet.

Eine reaktionsfähige, veresterte Hydroxygruppe in den genannten Verfahrensvarianten ist beispielsweise eine Hydroxygruppe, welche durch eine starke Säure, insbesondere eine starke anorganische Säure wie Halogenwasserstoff, z.B. Chlor-, Brom- oder Jodwasserstoff, oder Schwefelsäure, oder durch eine starke organische Säure, beispielsweise eine Sulfonsäure, wie eine aliphatische oder aromatische Sulfonsäure, z.B. Methansulfonsäure, p-Toluolsulfonsäure oder 4-Bromobenzolsulfonsäure, verestert ist. Eine solche reaktionsfähige, veresterte Hydroxygruppe ist insbesondere Halogen, z.B. Chlor, Brom oder Jod, oder durch aliphatische oder aromatische Gruppen substituiertes Sulfonyloxy, z.B. Mesyloxy oder Tosyloxy.

$R_3$ mit der Bedeutung "veräthertes Hydroxy" in der Verfahrensvariante c) ist beispielsweise $C_1$-$C_4$-Alkoxy, z.B. Methoxy oder Ethoxy, am Phenylring gegebenenfalls durch diverse Substituenten wie $C_1$-$C_4$-Alkyl, z.B. Methyl, Halogen, z.B. Chlor, oder $C_1$-$C_4$-Alkoxy, z.B. Methoxy, substituiertes Benzyloxy oder Pyridyl-$C_1$-$C_4$-alkoxy, z.B. Pyridylmethoxy.

Die in den Ausgangsmaterialien, Zwischenstufen und in Verbindungen der Formel IA vorhandenen funktionellen Gruppen, insbesondere die Carboxy-, Amino-, Hydroxy- und Sulfogruppen, sind gegebenenfalls durch solche Schutzgruppen (conventional protecting groups) geschätzt, die üblicherweise in der Penicillin-, Cephalosporin- und Peptidchemie verwendet werden. Diese Schutzgruppen sollen die betreffenden funktionellen Gruppen gegen unerwünschte Nebenreaktionen wie Acylierungen, Verätherungen, Veresterungen, Oxydationen, Solvolyse etc. schützen und unter schonenden Reaktionsbedingungen leicht abspaltbar sein, das heisst unter Vermeidung von Spaltung z.B. solvolytischer, reduktiver, photolytischer oder auch enzymatischer Spaltung, sowie anderer Nebenreaktionen.

Der Schutz von funktionellen Gruppen mit solchen Schutzgruppen, die Schutzgruppen selbst, sowie ihre Abspaltungsreaktionen, sind beispielsweise in "Protective Groups in Organic Chemistry", Plenum Press, London und New York 1973, in Greene, Th.W. "Protective Groups in Organic Synthesis", Wiley, New York 1981, in "The Peptides", Vol. I, Schröder and Lubke, Academic Press, London und New York 1965, sowie in "Methoden der organischen Chemie", Houben-Weyl, 4. Auflage, Bd. 15/I Georg Thieme Verlag, Stuttgart 1974, beschrieben.

Die Herstellung von Verbindungen der Formel IA gemäss Verfahrensvariante a) kann man analog bekannten N-Alkylierungsreaktionen durchführen.

Die Herstellung von Verbindungen der Formel IA durch reduktive N-Alkylierung von Ausgangsmaterialien, worin X Oxo bedeutet, kann man analog bekannten Reaktionsbedingungen, z.B. mit Hydridreduktionsmitteln wie Natriumcyanborhydrid, durchführen. Die reduktive Aminierung mit Hydridreduktionsmitteln wird vorzugsweise in inertem Lösungsmittel wie Methanol oder Acetonitril, bevorzugt in Gegenwart einer Säure wie Chlorwasserstoffsäure oder Essigsäure, durchgeführt.

Die Herstellung von Verbindungen der Formel IA gemäss Verfahrensvariante a) aus Augsgangsmaterialien, worin X eine reaktionsfähige, veresterte Hydroxygruppe und gleichzeitig Wasserstoff darstellt, führt man gegebenenfalls in Gegenwart von Basen wie Triethylamin oder Kaliumcarbonat in einem inerten Lösungsmittel analog bekannten Reaktionsbedingungen für N-Alkylierungen durch.

Die Herstellung von Verbindungen der Formel IA gemäss Verfahrensvariante b) wird unter den für die Verfahrensvariante a) genannten Reaktionsbedingungen durchgeführt, beispielsweise bei N-Alkylierungen, wenn das Ausgangsmaterial ein reaktiver Ester eines Alkohols der Formel $R_1$-OH oder $R_2$-OH ist, oder N-Alkylierungen unter reduzierenden Bedingungen, wenn das Ausgangsmaterial ein Aldehyd, z.B. ein $C_1$-$C_4$-Alkanal oder ein Aryl-$C_1$-$C_4$-alkanal ist.

Die Herstellung von Verbindungen der Formel IA, worin $R_3$ Hydroxy ist, gemäss Verfahrensvariante c) aus Ausgangsmaterialien der Formel VI, worin $R_3$ veräthertes Hydroxy darstellt, erfolgt in an sich bekannter Weise, z.B. in Gegenwart einer Mineralsäure wie Jodwasserstoff, und zwar bevorzugt aus solchen Ausgangsmaterialien, worin $R_3$ $C_1$-$C_4$-Alkoxy, z.B. Methoxy, ist, durch Umsetzung mit Natrium- oder Lithiumdiphenylphosphid in Tetrahydrofuran unter Rückflusstemperatur oder mit Bortribromid in Methylenchlorid.

Bei Verwendung von Ausgangsmaterialien der Formel VI, worin Z Sauerstoff und $R_3$ gegebenenfalls substituiertes Benzyloxy bedeuten, führt man die Herstellung der Endstoffe gemäss Verfahrensvariante c) vorzugsweise unter den bekannten Bedingungen der katalytischen Hydrierung, beispielsweise mit Palladium als Katalysator durch.

Die Herstellung von Verbindungen der Formel IA gemäss Verfahrensvariante d) führt man unter solchen Reaktionsbedingungen durch, welche für Reaktionen, in denen Kohlenstoff-Stickstoff-Doppelbindungen oder Kohlenstoff-Kohlenstoff-Doppelbindungen in einer Enamin-Gruppierung gesättigt werden, bekannt sind, z.B. durch Umsetzung mit Hydridreduktionsmitteln wie Lithiumaluminiumhydrid oder einem Boran, Natriumborhydrid, Natriumcyanoborhydrid oder Diboran, in inerten Lösungsmitteln wie THF oder Diethylether, vorzugsweise bei Raumtemperatur oder unter Erwärmen.

Ausgangsmaterialien der Formel II sind bekannt oder können nach bekannten Verfahren hergestellt werden.

Ausgangsmaterialien der Formel II, worin Z Schwefel bedeutet, d.h. die gegebenenfalls substituierten 3,4-Dihydro-2H-[1]-benzothiopyran-3-one (Thiochroman-3-one) können nach an sich bekannten Verfahren hergestellt werden, z.B. wie in J. Org. Chem. 34, 1566 (1969) oder in den Beispielen beschrieben.

Ausgangsmaterialien der Formel II, worin Z Sauerstoff bedeutet, d.h. die Chroman-3-one, können nach an sich bekannten Verfahren hergestellt werden, z.B. wie in J. Chem. Soc. 1610 (1948), J. Amer. Chem. Soc. 87, 3958 (1965) und im Bull Soc. Chim. Belg. 82, 283 (1973) oder wie in den Bespielen beschrieben.

Beispielsweise wird gemäss der in Bull. Soc. Chim. Belg. 82, 283 (1973) beschriebenen Methode ein durch $C_1$-$C_4$-Alkoxy oder Benzyloxy substituiertes Salicylaldehyd oder Thiosalicylaldehyd mit einem Acrylsäure-$C_1$-$C_4$-alkylester kondensiert und das entsprechend substituierte 2H-[1]-(Benzopyran- oder Benzothiopyran)-3-carbonsäure-Derivat erhalten. Curtius-Abbau zum 3-Amino-2H-[1]-(benzopyran oder -benzothiopyran)-Derivat und saure Hydrolyse ergibt die entsprechend substituierten Chroman-3-on oder Thiochroman-3-on-Derivate.

Ausgangsmaterialien der Formel II, insbesondere solche, worin Z Sauerstoff darstellt, können in vorteilhafter Weise gemäss folgendem neuen Verfahren wie folgt hergestellt werden:

Ein durch $C_1$-$C_4$-Alkoxy oder Benzyloxy substituiertes 2H-[1]-benzo-pyran, z.B. eine Verbindung wie in J. Org. Chem. 38, 3832 (1973) beschrieben, wird mit einem Halogenierungsmittel, z.B. mit N-Bromosuccinimid oder mit N-Bromoacetamid, in einem Hydroxylgruppenhaltigen Lösungsmittel, z.B. in wässrigem Aceton, umgesetzt, worauf man das entsprechend substituierte 3-Halo-4-hydroxychroman erhält. Durch Umsetzung mit einer nicht-wässrigen Base, z.B. Natriumhydrid in Tetrahydrofuran, erhält man das entsprechend substituierte Chroman-3,4-epoxid. Nach Umlagerung in einem Lewis-Säure-haltigen Reagenz, z.B. Zinkjodid in Toluol, erhält man die entsprechend durch Niederalkoxy oder Benzyloxy substiutierte Zwischenstufe.

Die Ausgangsmaterialien der Formel II, worin Z Schwefel bedeutet, können in die Mono- oder Di-S-Oxide durch Behandlung mit einer Peroxogruppen-haltigen Säure, z.B. Metachlorperbenzoesäure, übergeführt werden, wobei man in Abhängigkeit von der eingesetzten molaren Menge an Persäure das Mono-S-Oxid (1 Aequivalent) oder das Di-S-Oxid (2 und mehr Aequivalente) erhält. Sulfoxide lassen sich auch durch Umsetzung mit Perjodsäuresalzen, z.B. Natriumperjodat, herstellen.

Die Hydroxy-substituierten Chroman-3-one und Thiochroman-3-one werden vorzugsweise durch Etherspaltung der entsprechenden Niederalkylether oder Benzylether, vorzugsweise durch Spaltung der Methylether mit für Etherspaltungen bekannten Reagenzien, insbesondere mit Lithiumdiphenylphosphid in Tetrahydrofuran unter Rückflussbedingungen, erhalten.

Die durch Acyloxygruppen substituierten 3,4-Dihydro-2H-[1]-(benzopyran-3-one und benzothiopyran)-3-one können durch Acylierung der entsprechenden Hydroxy-substituierten 3,4-Dihydro-2H-[1]-(benzopyran-3-one und benzothiopyran-3-one) analog bekannten Acylierungsverfahren hergestellt werden.

Die primären und sekundären 3-Amino-3,4-dihydro-2H-[1]-(benzopyran und benzothiopyran)-Ausgangsmaterialien der Formel IV können aus Ausgangsmaterialien der Formel II mit der Bedeutung X = Oxo durch Umsetzung mit Ammoniak, einem primären Amin der Formel $R_1NH_2$ oder $R_2NH_2$ oder vorzugsweise mit einem Säureadditionssalz davon in Gegenwart eines Reduktionsmittels, vorzugsweise mit Natriumcyanborhydrid hergestellt werden.

Primäre 3-Amino-3,4-dihydro-2H-[1]-(benzopyran und benzothiopyran)-Ausgangsmaterialien der Formel IV, welche für die Verfahrensvariante b) verwendbar sind, können ebenfalls durch Umsetzung eines gegebenenfalls substituierten Phenol- oder Thiophenolderivats mit Alpha-Halomethylacrylsäure und anschliessender Zyklisierung der Alpha-Phenoxymethyl- oder -Phenylthiomethylacrylsäure bei erhöhter Temperatur zur gegebenenfalls substituierten 3,4-Dihydro-2H-[1]-(benzopyran oder benzothiopyran)-3-carbonsäure hergestellt werden. Nach Curtius-Abbau durch Umsetzung mit Diphenylphosphonylazid erhält man die entsprechend substituierten 3,4-Dihydro-3-amino-2H-[1]-(benzopyran oder benzothiopyran)-Derivate.

Die Zwischenprodukte der Formel VI, welche in der Verfahrensvariante c) zur Anwendung gelangen, lassen sich beispielsweise durch reduktive Aminierung von entsprechend substituierten 3,4-Dihydro-2H-[1]-(benzopyran und benzothiopyran)-3-on-Derivaten mit einem Amin der Formel III analog den weiter vorn beschriebenen Reaktionsbedingungen herstellen.

Zwischenprodukte der Formel VII können durch Behandlung der entsprechend substituierten 3,4-Dihydro-2H-[1]-(benzopyran und benzothiopyran)-3-one mit einem Amin der Formel III unter Reaktionsbedingungen, welche durch Wasserentzug gekennzeichnet sind, z.B. in Gegenwart von Molekularsieb, Bortrifluoridetherat oder p-Toluolsulfonäsure, in einem inerten Lösungsmittel wie Toluol oder Methylenchlorid hergestellt werden.

Zwischenprodukte der Formel VIII können durch Acylierung einer Verbindung der Formel IV, worin Y -$NHR_1$ oder -$NHR_2$ darstellt, mit einer Carbonsäure der Formel $R_7$-COOH, worin $R_7$ weiter vorn definiert ist, in Gegenwart eines gängigen Kondensationsmittels wie N,N-Dicyclohexylcarbodiimid oder durch Umsetzung mit einem reaktionsfähigen, funktionellen Säurederivat, z.B. dem Säurechlorid, oder einem gemischten Anhydrid, welches man durch Reaktion der Säure mit einem Halogenameisensäureester wie Chlorameisensäureethylester, in einem inerten, gegebenenfalls wasserfreien Lösungsmittel wie Methylenchlorid bildet, vorzugsweise in Gegenwart einer Base wie Pyridin, hergestellt werden.

Erfindungsgemässe Verbindungen, die nach irgendeiner der weiter vorn beschriebenen Verfahrensvarianten erhalten werden, können analog bekannten Verfahren, wie zum Beispiel im folgenden Abschnitt erläutert wird, in andere erfindungsgemässe Verbindungen der Formel I umgewandelt werden.

So kann die Umwandlung von Verbindungen der Formel I, worin $R_3$ Hydroxy bedeutet, in Verbindungen, worin $R_3$ Acyloxy bedeutet, z.B. in die $C_1$-$C_4$-Alkanoyloxy oder Aryloxyderivate, durch Umsetzung mit der entsprechenden freien Carbonsäure oder einem reaktionsfähigen, funktionellen Säurederivat analog bekannten Acylierungs- oder Veresterungsbedingungen erfolgen.

Die Umwandlung von Verbindungen der Formel I, worin $R_3$ Hydroxy bedeutet, in Verbindungen, worin $R_3$ $C_1$-$C_4$-Alkoxy oder Phenyl-$C_1$-$C_4$-alkoxy bedeutet, kann bevorzugt durch Kondensation mit der äquivalenten Menge des reaktionsfähigen Esters eines $C_1$-$C_4$-Alkanols oder Phenyl-$C_1$-$C_4$-Alkanols, z.B. einer Halogen-Verbindung, wie einem $C_1$-$C_4$-Alkyl- oder Phenyl-$C_1$-$C_4$-alkyliodid, in Gegenwart von äquivalenten Mengen von starken Basen wie Natriumhydrid in einem nichtwässrigen Lösungsmittel, wie Dimethylsulfoxid oder Dimethylformamid, erfolgen.

In allen obigen Reaktionen, kann es vorteilhaft sein, potentiell reaktionsfähige, z.B. die Hydroxy-, Amino- oder Carboxygruppe oder andere störende Gruppen, gemäss an sich bekannten Methoden zu schützen. Man schützt solche Gruppen vor der gewünschten Reaktion und, wenn erwünscht, spaltet nachträglich die Schutzgruppen ab, wobei man die gewünschten Verbindungen, z.B. solche der Formel I oder Zwischenprodukte erhält.

Eine Hydroxygruppe kann in Form von Estern, z.B. als Acyloxyderivate, z.B. als Niederalkanoyl-, Benzyloxycarbonyl- oder Niederalkoxycarbonylester, oder in Form von Ethern, z.B. als Tetrahydropyranyl- oder Benzylether, geschützt werden. In einer erhaltenen geschützten Verbindung der Formel I oder in einem Zwischenprodukt, worin eine oder mehrere funktionelle Gruppen geschützt sind, können die geschützten funktionellen Gruppen, z.B. die Hydroxy-, Amino- oder Carboxygruppen, nach an sich bekannten Methoden, z.B. Solvolyse, insbesondere Hydrolyse mit einer Säure, oder durch Reduktion, insbesondere Hydrogenolyse, freigesetzt werden.

Die oben genannten nachträglichen Reaktionen werden nach an sich bekannten Methoden, in Gegenwart oder Abwesenheit von Verdünnungsmitteln, vorzugsweise solche, welche gegenüber den Reagenzien inert sind und diese lösen, Katalysatoren, Kondensations- oder anderen oben genannten Mitteln, und/oder in einer inerten Atmosphäre, unter Kühlung, bei Zimmertemperatur oder bei erhöhter Temperatur, vorzugsweise beim Siedepunkt des verwendeten Lösungsmittels, bei normalem oder erhöhtem Druck, durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird. Immer wenn gewünscht, werden die oben genannten Verfahren erst durchgeführt, nachdem alle potentiell störenden, reaktionsfähigen funktionellen Gruppen in geeigneter Weise geschützt sind, z.B. wie oben und in den Beispielen illustriert.

In den genannten Reaktionen werden vorteilhafterweise solche Ausgangsstoffe vewendet, welche zu den weiter vorn als besonders bevorzugt beschriebenen Verbindungen führen.

Die Erfindung betrifft auch neue Ausgangsstoffe und Verfahren zu ihrer Herstellung.

Abhängig von den gewählten Ausgangsstoffen und Methoden können die neuen Verbindungen in Form von Isomeren oder als Isomerengemische vorliegen, zum Beispiel als geometrische Isomeren oder Mischungen davon (cis oder trans), als optische reine Antipoden, Racemate oder Diastereomeren.

Erhaltene geometrische oder diastereomere Isomerengemsiche der obigen Verbindungen oder Zwischenprodukte können nach an sich bekannten Methoden in die einzelnen racemischen oder optisch aktiven Isomeren getrennt werden, z.B. durch fraktionierte Destillation, Kristallisation und/oder Chromatographie. Racemische Produkte können gleichfalls in die optischen Antipoden gespalten werden, z.B. durch Trennung ihrer diastereomeren Salze, wie gemäss J. Org. Chem. 43, 3803 (1978), z.B. durch fraktionierte Kristallisation von d- und ℓ-(Tartraten, Mandelaten, Camphersulfaten oder 1-Naphthyl-1-äthylisocyanaten) von Verbindungen, die eine basische, salzbildende Gruppe haben, oder von d- und ℓ-(α-Methylbenzylamin, Cinchonidin,, Chinin, Chinidin, Ephedrin, Dehydroabietylamin, Brucin oder Strychnin)-Salzen von Verbindungen, die eine saure,salzbildende Gruppe besitzen.

Bevorzugt wird jeweils das wirksamere Isomere und/oder der wirksamere Antipode der erfindungsgemässen Verbindungen isoliert.

Die Verbindungen der vorliegenden Erfindung werden entweder in freier oder in Form ihrer Salze erhalten. Jede erhaltene Base kann in das entsprechende Säureadditionssalz umgewandelt werden, vorzugsweise unter Verwendung einer therapeutisch annehmbaren Säure oder eines Anionenaustauschers. Erhaltene Salze können in die entsprechenden freien Basen umgewandelt werden, zum Beispiel unter Verwendung einer stärkeren Base, beispielsweise eines Metall-oder Ammoniumhydroxids oder eines basischen Salzes, z.B. eines Alkalimetallhydroxids oder -carbonats, oder eines Kationenaustauschers. Diese oder andere Salze, etwa Pikrate, können auch zur Reinigung der erhaltenen Basen verwendet werden, indem man die Basen in Salze überführt, diese abtrennt und reinigt, und aus den Salzen wiederum die Base freisetzt. Infolge der engen Beziehung zwischen den freien Verbindungen und ihren Salzen sind in diesem Zusammenhang unter freien Verbindungen sinn- und zweckmässig gegebenenfalls immer auch die entsprechenden Salze zu verstehen.

Die Verbindungen und ihre Salze können auch in Form ihrer Hydrate erhalten werden, oder sie können andere zur Kristallisation verwendete Lösungsmittel einschliessen.

Die vorliegende Erfindung betrifft ebenfalls die Verwendung von Verbindungen der Formel I zur Herstellung von pharmazeutischen Zusammensetzungen, insbesondere pharmazeutischen Zusammensetzungen mit stimulierender Wirkung auf Serotoninrezeptoren, zum Gegenstand, welche sich beispielsweise für die Behandlung von Depression und Angstzuständen eignen.

Die erfindungsgemässen pharmazeutischen Präparate eignen sich zur enteralen, wie oralen oder rektalen, transdermalen oder parenteralen Verabreichung an Säugetieren, inkl. Menschen, zur Behandlung von Krankheiten, die in Folge von Serotoninrezeptorstimulierung, z.B. Depressionen und Angstzuständen, auftreten. Diese Präparate enthalten eine wirksame Menge einer Verbindung der Formel I oder eines pharmazeutisch annehmbaren Salzes davon und zwar ohne Beimengung oder in Kombination mit einem oder mehreren pharmazeutisch annehmbaren Trägermaterialien.

Die pharmakologisch aktiven Verbindungen der Erfindung sind bei der Herstellung von pharmazeutischen Zusammensetzungen verwendbar, die eine wirksame Menge derselben in Verbindung oder Beimischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z.B. Lactose, Dextrose, Sucrose, Mannit, Sorbit, Cellulose und/oder Glycin, b) Gleitmitteln, z.B. Siliciumdioxid, Talk, Stearinsäure, deren Magnesium- oder Calciumsalz und/oder Polyethylenglycol, für Tabletten auch c) Bindemitteln, z.B. Magnesiumaluminiumsilicat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z.B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare Präparate sind vorzugsweise wässrige isotonische Lösungen oder Suspensionen, und Suppositorien werden vorteilhaft aus Fettemulsionen oder -suspensionen hergestellt. Diese Zusammensetzungen können sterilisiert werden und/oder Adjuvanzien, wie Konservierungs-, Stabilisierungs-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier- bzw. Ueberzugsmethoden hergestellt und enthalten etwa 0,1 bis 75 %, vorzugsweise etwa 1 bis 50 %, des aktiven Bestandteils.

Geeignete Formulierungen für die transdermale Anwendung enthalten eine wirksame Menge einer Verbindung der Formel I zusammen mit Trägermaterial. Geeignete Trägermaterialien enthalten pharmazeutisch annehmbare Hilfsstoffe, welche den Durchgang durch die Haut ermöglichen. Insbesondere haben transdermale therapeutische Systeme die Form eines Pflasters mit einer Schutzschicht, einem Wirkstoffreservoir, gegebenenfalls mit Trägermaterialien, und einer die Abgabe bestimmenden Kontrollschicht, durch welche der Wirkstoff auf die Haupt mit kontrollierter und bestimmbarer Geschwindigkeit über einen längeren Zeitabschnitt abgegeben wird. Das System hat gegebenenfalls noch eine Klebschicht.

Verbindungen der Formel I mit stimulierenden Eigenschaften auf präsynaptische Dopaminrezeptoren und pharmazeutische Zusammensetzungen mit den erfindungsgemässen Verbindungen sind geeignet zur Behandlung von Säugetieren inkl. Menschen bei Störungen des zentralen Nervensystems als Folge von Stimulierungserscheinungen von präsynaptischen Dopaminrezeptoren, insbesondere von psychotischen Zuständen, z.B. Schizophrenie, Parkinsonismus und Dyskinesie.

Die applizierbare Wirkstoffdosis ist speziesabhängig sowie vom Körpergewicht, dem Alter und individuellen Zustand und der Darreichungsform abhängig.

Die Einheitsdosis für einen Säuger, z.B. Mensch, von ca. 50 bis 70 kg Gewicht beträgt zwischen 15 und 200 mg des Wirkstoffs.

Die folgenden Beispiele sollen die Erfindung erläutern. Die Temperaturen sind in Celsiusgraden angegeben, und sämtliche Teile sind in Form von Gewichtsteilen angegeben. Wenn nicht anders erwähnt,

werden sämtliche Verdampfungen unter vermindertem Druck vorzugsweise zwischen etwa 15 und 100 mg Hg durchgeführt.

Beispiel 1:

a) Eine Mischung bestehend aus 4,6 g 3,4-Dihydro-5-methoxy-2H-[1]-benzothiopyran-3-amin, 12,9 g Natriumcarbonat und 21 ml n-Propyljodid in 40 ml Toluol und 40 ml Wasser wird unter Rückflussbedingungen und Rühren drei Tage lang erhitzt. Die organische Schicht wird getrocknet und das Lösungsmittel im Vakuum entfernt. Der Rückstand wird in Aether gelöst und mit einer äthanolischen Chlorwasserstofflösung angesäuert, worauf man 3,4-Dihydro-N,N-di-n-propyl-5-methoxy-2H-[1]-benzothiopyran-3-amin-hydrochlorid erhält. F.p.: 219-221°.

Das Ausgangsmaterial wird folgendermassen hergestellt: Eine gekühlte Mischung von 30,6 g m-Methoxythiophenol und 54,4 g 45%igem Kaliumhydroxid in 100 ml Dimethylsulfoxid wird mit 36,0 g alpha-Brommethylacrylsäure in 25 ml Dimethylsulfoxid so langsam versetzt, dass die Mischung die Reaktionstemperatur von ca. 50-55° nicht übersteigt. Nach einer Stunde Reaktionszeit wird das Reaktionsgemisch mit Wasser verdünnt und mit Aether gewaschen. Nach dem Ansäuern extrahiert man das Produkt mit Aether, trocknet die organische Schicht über Natriumsulfat und zieht das Lösungsmittel ab, worauf man alpha-3-Methoxythiophenylmethylacrylsäure erhält.

Dieses Zwischenprodukt wird in 570 ml o-Dichlorbenzol und 7,2 g Triäthylamin gelöst und bis 200° 5 Stunden lang erhitzt. Nach dem Abkühlen säuert man die wässrige Schicht an und extrahiert die Produkte mit Aether. Nach dem Trocknen über Magnesiumsulfat entfernt man das Lösungsmittel und erhält ein Gemisch bestehend aus 3,4-Dihydro-5-methoxy-2H-[1]-benzothiopyran-3-carbonsäure und 3,4-Dihydro-7-methoxy-2H-[1]-benzothiopyran-3-carbonsäure.

Das Gemisch dieser beiden Säuren wird in 500 ml tert-Butanol gelöst und mit 17 g Triäthylamin und 36 ml Diphenylphosphorylazid umgesetzt. Nach 5 Stunden langem Erhitzen unter Rückfluss wird das Lösungsmittel im Vakuum entfernt, und der Rückstand in Aether aufgenommen und mit 1N wässriger Natriumhydroxidlösung und 1N wässriger Chlorwasserstofflösung gewaschen. Nach dem Trocknen über Magenesiumsulfat wird das Lösungsmittel im Vakuum entfernt und der Rückstand auf Silicagel mit einem Aether-Hexan-1:4-Gemisch chromatographiert, worauf man nacheinander N-tert-butoxycarbonyl-3,4-dihydro-5-methoxy-2H-[1]-benzothiopyran-3-amin und N-tert-Butoxycarbonyl-3,4-dihydro-7-methoxy-2H[1]-benzothiopyran-3-amin erhält.

Eine Lösung von 10 g N-tert-Butoxycarbonyl-3-4-dihydro-5-methoxy-2H-[1]-benzhothiopyran-3-amin in 30 ml Trifluoressigsäure lässt man bei Raumtemperatur eine Stunde lang reagieren. Das Lösungsmittel wird im Vakuum entfernt und der Rückstand mit 1N wässriger Natrium-hydroxid-Lösung behandelt. Nach Extrahieren mit Aether, Trocknen über Magnesiumsulfat und Entfernen des Lösungsmittels erhält man 3,4-Dihydro-5-methoxy-2H-[1]-benzothiopyran-3-amin als Oel.

Beispiel 2:

a) Zu einer Lösung bestehend aus 2,9 ml Diphenylphosphin und 7,4 ml 2,2 M n-Butyllithium in 17 ml trockenem Tetrahydrofuran werden 2,5 g N,N-Dipropyl-5-methoxy-3,4-dihydro-2H-[1]-benzothiopyran-3-amin gegeben. Nach zwei Stunden langem Erhitzen unter Rückflussbedingungen wird das Reaktionsgemisch neutralisiert, mit Aether verdünnt und mit Wasser gewaschen. Nach dem Trocknen über Magnesiumsulfat wird das Lösungsmittel im Vakuum entfernt, der Rückstand in Aether aufgelöst und mit wässriger Chlorwasserstoffsäure angesäuert, worauf man 3,4-Dihydro-N,N-dipropyl-5-hydroxy-2N-[1]-benzothiopyran-3-aminhydrochlorid erhält. F.p.: 223-226°.

Beispiel 3

a) Zu einer Lösung von 220 mg 3,4-Dihydro-N,N-dipropyl-5-hydroxy-2H-[1]-benzothiopyran in 2 ml Dimethylsulfoxid werden 100 mg 50%ige Natriumhydrid-Suspension und 150 mg Ethyliodid gegeben. Nach einer Stunde Reaktionszeit bei 50° wird das Reaktionsgemisch mit Wasser verdünnt und anschliessend mit Aether extrahiert. Nach Trocknen über Magnesiumsulfat wird das Lösungsmittel im Vakuum entfernt und der Rückstand mit äthanolischer Salzsäurelösung angesäuert. Nach Verdünnen mit Aether erhält man 3,4-Dihydro-N,N-dipropyl-5-ethoxy-2H-[1]-benzothiopyran-3-amin-hydrochlorid. F.p.: 168-174°.

Analog werden folgende Verbindungen erhalten:
b) 3,4-Dihydro-N,N-dipropyl-5-propyloxy-2H-[1]-benzothiopyran-3-amin-hydrochlorid, F.p. 156-161°;
c) 3,4-Dihydro-N,N-dipropyl-5-benzyloxy-2H-[1]-benzothiopyran-3-amin-hydrochlorid.

Beispiel 4:

a) Eine Mischung aus 500 mg 3,4-Dihydro-5-methoxy-2H-[1]-benzothiopyran-3-amin und 361 mg Essigsäureanhydrid in 10 ml Methylenchlorid wird 15 Minuten lang bei Raumtemperatur stehengelassen. Man entfernt das Lösungsmittel im Vakuum und erhält die 3,4-Dihydro-N-acetyl-5-methoxy-2H-[1]-benzopyran-3-amin-Verbindung, welche man in 10 ml 1M Diboran-Lösung in Tetrahydrofuran auflöst. Nach 3 Stunden langem Erwärmen unter Rückflussbedingungen wird das Reaktionsgemisch auf 6N Chlorwasserstoffsäure gegossen, mit Wasser gewaschen, mit 45%iger wässriger Kaliumhydroxid-Lösung basisch gemacht und mit Aether wiederum extrahiert. Nach Trocknen über Magnesiumsulfat wird das Lösungsmittel abgezogen. Nach Ansäuern mit äthanolischer Chlorwasserstofflösung erhält man 3,4-Dihydro-N-ethyl-5-methoxy-2H-[1]-benzothiopyran-3-amin-hydrochlorid, F.p. 222-224°.
b) Analog wird 3,4-Dihydro-N-ethyl-5-methoxy-7-methyl-2H-[1]-benzothiopyran-3-amin-hydrochlorid, F.p. 250-252° hergestellt.

Beispiel 5

a) Eine Mischung bestehend aus 4,0 g 5-Methoxy-3,4-dihydro-2H-[1]-benzopyran-3-on, 30 ml N,N-Di-n-propylamin, 200 ml Toluol und 0,5 ml Trifluoressigsäure wird 3 Stunden in einer Dean-Stark-Apparatur erhitzt. Das Lösungsmittel wird im Vakuum entfernt und der Rückstand zu einer Lösung von 7 g Natriumcyanoborhydrid in 160 ml Aethanol und 40 ml Essigsäure gegeben. Nach 15 Minuten Reaktionszeit bei Raumtemperatur wird das Reaktionsgmemisch mit 6N wässriger Chlorwasserstoffsäure verdünnt, mit Aether gewaschen, mit 45%iger wässriger Kaliumhydroxidlösung basisch gemacht und mit Aether wiederum extrahiert. Nach dem Trocknen über Magnesiumsulfat wird das Lösungsmittel im Vakuum entfernt. Nach Zugabe von äthanolischer Chlorwasserstofflösung zu dem in Aether aufgenommenen Rückstand erhält man 5-Methoxy-3,4-dihydro-N,N-dipropyl-2H-[1]-benzothiopyran-3-amin-hydrochlorid mit einem Schmelzpunkt von 219-221°.

Das Ausgangsmaterial, 3,4-Dihydro-5-methoxy-2H-[1]-benzothiopyran-3-on, kann wie in Beispiel 1 beschrieben aus 3,4-Dihydro-5-methoxy-2H-[1]-benzothiopyran-3-carbonsäure erhalten werden.

Das Ausgangsmaterial kann auch folgendermaßen erhalten werden: Zu einer gut gerührten Lösung von 36,4 g 5-Methoxy-2H-[1]-benzopyran in 300 ml Aceton und 100 ml Wasser werden 29 g N-Bromsuccinimid in Portionen über einen Zeitraum von 5 Minuten gegeben. Nach 4 Stunden Reaktionszeit wird das Reaktionsgemisch mit Wasser verdünnt und mit Aether extrahiert. Die vereinigten organischen Phasen werden mit Wasser gewaschen und über Magnesiumsulfat getrocknet. Nach Abziehen des Lösungsmittel wird der Rückstand mit Aether-Hexan verrieben, worauf man trans-3-Brom-4-hydroxy-5-methoxy-3,4-dihydro-2H-[1]-benzopyran erhält.

Zu einer Suspension von 5,0 g Natriumhydrid in 100 ml trockenem Tetrahydrofuran wird tropfenweise unter Rühren eine Lösung von 13 g trans-3-Brom-4-hydroxy-5-methoxy-3,4-dihydro-2H-[1]-benzopyran in 150 ml Tetrahydrofuran gegeben. Nach 30 Minuten Rühren bei Raumtemperatur wird das Reaktionsgemisch filtriert und das Lösungsmittel abgezogen. Nach Lösen des Rückstands in 150 ml Toluol werden 1,0 g wasserfreies Zinkjodid hinzugefügt und 15 Minuten unter Rückflussbedingungen erhitzt. Das Reaktionsgemisch wird durch 120 g Silicagel mit Methylenchlorid als Elutionsmittel filtriert. Nach Abziehen des Lösungsmittels erhält man 5-Methoxy-3,4-dihydro-2H-[1]-benzopyran-3-on (5-Methoxychroman-3-on) als Oel.

Beispiel 6:

Zu einer Lösung von 8,55 ml Diphenylphosphin in 50 ml trockenem Tetrahydrofuran werden 22 ml 2,2M n-Butyllithium in Hexan bei 0° gegeben. Zu dieser Lösung gibt man etwas Tetrahydrofuran und erhitzt vier Stunden lang unter Rückflussbedingungen. Man verdünnt das Reaktionsgemisch mit Aether und extrahiert mit 3N Salzsäure. Die wässrige Phase wird mit Aether gewaschen, mit wässriger Natriumhydrogencarbonat-Lösung neutralisiert und mit Methylenchlorid extrahiert. Nach Trocknen über Magnesiumsulfat zieht man das Lösungsmittel ab und löst den Rückstand in einem Acetonitril-Aether-Gemisch. Nach Ansäuern mit 3N Salzsäure-Lösung erhält man 5-Hydroxy-3,4-dihydro-N,N-dipropyl-2H-[1]-benzopyran-3-amin-hydrochlorid. F.p.: 206-210°.

Beispiel 7:

a) Zu einer Suspension bestehend aus 55 mg Natriumhydrid in 10 ml Dimethylsulfoxid werden 290 mg 5-Hydroxy-N,N-dipropyl-3,4-dihydro-2H-[1]-benzopyran-3-amin gegeben. Nach 15 Minuten Reaktionszeit werden 180 mg Aethyljodid hinzugefügt und drei Stunden lang gerührt. Man entfernt das Lösungsmittel und verdünnt mit Wasser. Man extrahiert mit Aether und trocknet die organischen Phasen über Magnesiumsulfat. Nach Abziehen des Lösungsmittels wird der Rückstand in Aether aufgenommen und mit 6N äthanolischer Chlorwasserstoff-Lösung angesäuert, worauf man 5-Aethoxy-N,N-dipropyl-3,4-dihydro-2H-[1]-benzopyran-3-amin-hydrochlorid erhält. F.p.: 135-137°. Analog werden folgende Verbindungen hergestellt:
b) 5-Propyloxy-N,N-dipropyl-3,4-dihydro-2H-[1]-benzopyran-3-aminhydrochlorid, F.p.: 127-128°;
c) 5-Aethoxy-N,N-diethyl-3,4-dihydro-2H-[1]-benzopyran-3-aminhydrochlorid;
d) 5-Benzyloxy-N,N-dipropyl-3,4-dihydro-2H-[1]-benzopyran-3-aminhydrochlorid.

Beispiel 8:

a) Zu einer Lösung von 2,86 g 5-Hydroxy-3,4-dihydro-N,N-di-n-propyl-2H-[1]-benzopyran-3-amin-hydrochlorid in 40 ml Methylenchlorid werden 3,9 g Diisopropyläthylamin und 1,6 g Pivaloylchlorid hinzugefügt. Man rührt die Mischung 16 Stunden lang. Nach Verdünnen mit Methylenchlorid wird das Reaktionsgemisch mit Wasser gewaschen und mit Natriumhydrogencarbonat-Lösung gesättigt. Die organische Phase wird über Magnesiumsulfat getrocknet und das Lösungsmittel abgezogen. Nach Zugaben von Bromwasserstoff-Lösung in Isopropanol fällt 5-Trimethylacetoxy-3,4-dihydro-N,N-dipropyl-2H-[1]-benzopyran-3-hydrobromid aus. F.p. 130-139°.

Analog werden folgende Verbindungen hergestellt:
b) 5-Acetoxy-3,4-dihydro-N,N-dipropyl-2H-[1]-benzopyran-3-amin;
c) 5-Dimethylcarbamoyloxy-3,4-dihydro-N,N-dipropyl-2H-[1]-benzopyran-3-amin;
d) 5-Benzoyloxy-3,4-dihydro-N,N-dipropyl-2H-[1]-benzopyran-3-amin;
e) 5-Trimethylacetoxy-3,4-dihydro-N,N-dipropyl-2H-[1]-benzothiopyran-3-amin;
f) 5-Dimethylcarbamoyloxy-3,4-dihydro-N,N-dipropyl-2H-[1]-benzothiopyran-3-amin.

Beispiel 9:

Zu einer Lösung von 100 g 5-Aethoxy-3,4-dihydro-2H-[1]-benzothiopyran-3-on in 900 ml Toluol werden 975 g (192 ml) Di-n-propylamin und 220 mg Dichloressigsäure hinzugefügt. Man erhitzt die Lösung unter Rückflussbedingungen und entfernt das sich bildende Wasser in einer Dean-Stark Apparatur.Nach mehrstündigem Erhitzten unter Rückflussbedingungen und Entfernen des Lösungsmittels erhält man das Gemisch aus N,N-Dipropyl-5-äthoxy-2H-[1]-benzothiopyran-3-amin und N,N-Dipropyl-5-ethoxy-4H-[1-benzothiopyran-3-amin als Oel.

Zu 137 g Gemisch der beiden Enamine in 308 ml Tetrahydrofuran werden 32,6 g Natriumcyanoborhydrid in 308 ml Tetrahydrofuran gegeben. Man kühlt auf 5° und fügt 300 ml Essigsäure so hinzu, dass die Temperatur 5° nicht übersteigt. Man lässt bei Raumtemperatur über Nacht stehen, zieht das Lösungsmittel ab und löst den Rückstand in einem Gemisch aus 500 ml Aether und 500 ml Wasser. Die Mischung wird mit wässriger konzentrierter Ammoniak-Lösung basisch gemacht. Nach Waschen der wässrigen Phase werden die kombinierten Aetherphasen mit 6N Salzsäure gewaschen und anschliessend mit konzentrierter Ammoniak-Lösung wider basisch gemacht. Nach Extrahieren der wässrigen Phase mit Aether werden die organischen Phasen getrocknet und filtriert. Nach Abdampfen des Lösungsmittels erhält man ein schwarzes Oel, welches man durch Destillation (K.p. 160-165°, 0,6 mm Hg) reinigt. Das Amin wird in Aether gelöst und durch diese Lösung Chlorwasserstoffgas geblasen. Es kristallisiert das Hydrochlorid des Amins aus und man erhält nach Umkristallisieren aus Wasser 3,4-Dihydro-N,N-dipropyl-5-ethoxy-2H-[1]-benzothiopyran-3-amin-hydrochlorid, F.p.:174-175°.

Das Ausgangsmaterial wird folgendermassen hergestellt:
1,53 kg alpha-2,6-Dioxocyclohexylessigsäureäthylester in 7,7 1 Methylenchlorid werden unter Rückflussbedingungen erhitzt und dazu werden tropfenweise 1,75 Stunden lang 1,8 kg Thionylchlorid hinzugefügt. Die

Lösung wird 3 Stunden lang unter Rückflussbedingungen erhitzt. Man zieht das Lösungsmittel ab und destilliert den öligen Rückstand, worauf man alpha-2-Chlor-6-oxocyclohex-1-enylessigsäure-äthylester als gelbes Oel erhält. K.p.: 115-125° /0,2 mm.

Zu der Lösung von 700 g (3,23M) alpha-2-Chlor-6-oxocyclohex-1-enylessigsäureäthylester in 2,1 1 wasserfreiem Aethanol werden 410 g Mercaptoessigsäureäthylester hinzugefügt. Aschliessend fügt man bei 5° eine Lösung von 301 g Kalium-tert-butylat in 1,8 1 wasserfreiem Aethanol tropfenweise 1,5 Stunden lang hinzu. Man rührt weitere 2 Stunden bei dieser Temperatur und lässt anschliessend über Nacht stehen. Man zieht das Lösungsmittel ab und setzt 3 1 Aether und Wasser hinzu. Die organische Phase wird mit Wasser gewaschen, über Magnesiumsulfat getrocknet und das Lösungsmittel abgedampft, worauf man alpha-(2-Aethoxycarbonylmethylthio-6-oxo-cyclohex-1-enyl)-essigsäureäthylester als hellgelbes Oel erhält.

Zu einer Lösung von 156 g α-(2-Aethoxycarbonylmethylthio-6-oxocyclohex-1-enyl)essigsäureäthylester werden 500 ml Essigsäure gegeben. Nach Beendigung der Gasentwicklung wird das Lösungsmittel abgedampft und ein Oel erhalten, welches man mit Aether und Wasser vermischt. Die organische Phase wird mit Wasser gewaschen, mit wässriger Natiumhydrogencarbonat-Lösung neutralisiert und über Magnesiumsulfat getrocknet. Nach Abdampfen des Lösungsmittels erhält man 6-Acetoxy-2-ethoxycarbonylmethylthiophenylessigsäureäthylester als Oel.

132 g 6-Acetoxy-2-äthoxycarbonylmethylthiophenylessigsäureäthylester werden bei Raumtempertur zu einer Lösung von 34 g Ammoniak in 1,5 1 wasserfreiem Aethanol gegeben. Man lässt zwei Stunden stehen, dampft ab und nimmt den Rückstand in Methylenchlorid und Salzsäure auf. Man wäscht die organische Phase, neutralisiert mit wässriger Bicarbonat-Lösung, trocknet über Magnesiumsulfat und dampft ab, worauf man 6-Hydroxy-2-äthoxycarbonylthiomethylphenylessigsäureäthylester als ölige Flüssigkeit erhält.

Zu einer Lösung von 20 g 6-Hydroxy-2-äthoxycarbonylthiomethylphenylessigsäureäthylester in 200 ml wasserfreiem Aethanol werden 10,3 g Diäthylsulfat gegeben. Anschliessend fügt man portionsweise 7,53 g Kalium-tert-butylat hinzu und hält die Temperatur unterhalb 30°. Man rührt mehrere Stunden bei Raumtemperatur, dampft ab und versetzt den Rückstand mit Aether und Wasser. Die organische Phase wird mit 40 ml 0.05N eiskalter Nariumhydroxid-Lösung gewaschen, mit Wasser versetzt und getrocknet. Nach Abdampfen erhält man 6-Aethoxy-2-äthoxycarbonylethylthiophenylessigsäureäthylester als ölige Flüssigkeit.

Zu einer Lösung von 17,8 g 6-Aethoxy-2-äthoxycarbonylthiomethylphenylessigsäureäthylester in 112 ml Aethanol wird eine Lösung von 7,21 g Kaliumhydroxid in 28 ml Wasser gegeben. Man erhitzt die Mischung zwei Stunden lang unter Rückfluss, engt ein und verdünnt mit 200 ml Wasser. Nach Hinzufügen von 10 ml 12N Salzssäure kristallisiert das zuerst sich als Oel bildende Produkt aus, und man erhält die 6-Aethoxy-2-carboxymethylthioessigsäure mit einem Schmelzpunkt von 147-150°.

Zu einer Suspension von 12,4 g der Dicarbonsäure in 115 ml Essigsäureanhydrid gibt man 10,6 g wasserfreies Natriumacetat. Nach 20 Minuten langem Erhitzen des Reaktionsgemisches unter Rückflussbedingungen (Gasentwicklung) dampt man ab und versetzt den Rückstand mit Aether und wässriger Natriumhydrogencarbonat-Lösung. Nach Waschen der Aether-Phase und Trocknen über Magnesiumsulfat dampft man ein und destilliert den Rückstand, worauf man 3-Acetoxy-5-äthoxy-2H-[1]-benzothiopyran erhält. K.p. 155-163° (0,25 mm).

16,3 g des Enolacetats in 147 ml Aethanol werden zu einer Lösung von 149 ml 12N Salzsäure-Lösung gegeben. Man verdünnt mit etwas Wasser, erhitzt unter Rückfluss und dampft ein. Der Rückstand wird mit Aether extrahiert. Die Aetherphase wird mit gesättigter, wässriger Natriumbicarbonat-Lösung neutralisiert, gewaschen, mit Magnesiumsulfat getrocknet und eingedampft, worauf man 5-Aethoxy-3,4-dihydro-2H-[1]-benzothiopyran-3-on als Oel erhält.

Beispiel 10:

a) Herstellung von 10.000 Tabletten mit 10,0 mg Wirkstoff:

| Bestandteile | |
|---|---|
| 3,4-Dihydro-N,N-dipropyl-5-ethoxy-2H-[1]-benzothiopyran-3-amin-hydrochlorid | 200,00 g |
| Lactose | 2.400,00 g |
| Maisstärke | 125,00 g |
| Polyethylenglycol 6,000 | 150,00 g |
| Magnesiumstearat | 40,00 g |
| Wasser (steril) | q.s. |

Das pulverige Material wird durch ein Sieb mit Oeffnungen von 0,6 mm gedrückt. Anschliessend mischt man in einem Mischer den Wirkstoff mit der Lactose, dem Magnesiumstearat und der halben Menge Stärke. Die andere halbe Menge Stärke wird in 65 ml Wasser suspendiert und diese Suspension zu einer siedenden Lösung des Polyäthylenglycols 260 ml in Wasser gegeben. Diese gebildete Paste wird mit der Pulvermischung versetzt, welche gegebenenfalls mit weiterem Wasser granuliert wird. Man trocknet das Granulat bei 35°, treibt dieses durch ein Sieb mit 1,2 mm weiten Oeffnungen und komprimiert zu konkaven Tabletten mit oberer Bruchkerbe.

b) Herstellung von 1.000 Kapseln mit 10,0 mg Wirkstoff:

| Bestandteile | |
|---|---|
| 3,4-Dihydro-N,N-dipropyl-5-ethoxy-2H-[1]-benzothiopyran-3-amin-hydrochlorid | 10,00 g |
| Lactose | 207,00 g |
| Stärke | 80,00 g |
| Magnesiumstearat | 3,00 g |

Das pulverige Material wird durch ein Sieb mit Oeffnungen von 0,6 mm Durchmesser gedrückt. Anschliessend mischt man in einem Mischer den Wirkstoff mit der Lactose, dem Magnesiumstearat und der Stärke bis man eine homogene Masse erhält. Man füllt Nr. 2 Hartgelatinekapseln mit 300 mg der vorbereiteten Mischung.

Analog kann man Kapseln mit 5 - 100 mg eines anderen von der Formel I definierten Wirkstoffs herstellen.

## Ansprüche

1. Verbindungen der Formel

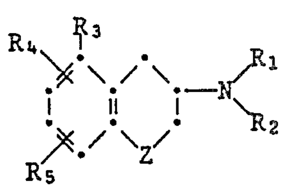

(I A),

worin Z Sauerstoff oder Schwefel, $R_1$ Wasserstoff, $C_1$-$C_4$-Alkyl, Benzyl oder 2-Phenylethyl, $R_2$ Wasserstoff, $C_1$-$C_4$-Alkyl, Benzyl oder 2-Phenylethyl oder $R_1$ und $R_2$ zusammengenommen Alkylen mit 4 bis 6 C-Atomen, $R_3$ Hydroxy, $C_1$-$C_4$-Alkoxy, Phenyl-$C_1$-$C_4$-alkoxy, welches am Phenylring durch 1-3 $C_1$-$C_4$-Alkyl, Halogen oder $C_1$-$C_4$-Alkoxy substituiert sein kann, $C_1$-$C_4$-Alkanoyloxy, Benzoyloxy, $C_1$-$C_4$-

Alkoxycarbonyloxy, Carbamoyloxy oder Mono- oder Di-$C_1$-$C_4$-alkylcarbamoyloxy und $R_4$ und $R_5$ unabhängig voneinander Wasserstoff, $C_1$-$C_4$-Alkyl oder Halogen bedeuten, Mono- oder Di-S-Oxide dieser Verbindungen, worin Z Schwefel bedeutet, und pharmazeutisch annehmbare Salze dieser Verbindungen.

**2.** Verbindungen gemäss Anspruch 1 der Formel I A, worin Z Sauerstoff bedeutet, und pharmazeutisch annehmbare Salze davon.

**3.** Verbindungen gemäss Anspruch 1 der Formel I A, worin Z Schwefel bedeutet, und pharmazeutisch annehmbare Salze davon.

**4.** Verbindungen gemäss Anspruch 1 der Formel I A, worin Z Sauerstoff oder Schwefel, $R_1$ Wasserstoff oder $C_1$-$C_4$-Alkyl, $R_2$ $C_1$-$C_4$-Alkyl, Benzyl oder 2-Phenylethyl oder $R_1$ und $R_2$ zusammen Butylen oder Pentylen, $R_3$ Hydroxy, $C_1$-$C_4$-Alkoxy, Benzyloxy, Acetoxy, Benzoyloxy, tert.-Butoxycarbonyloxy, Carbamoyloxy oder Mono- oder Di-$C_1$-$C_4$-alkylcarbamoyloxy, $R_4$ Wasserstoff, Halogen oder $C_1$-$C_4$-Alkyl und $R_5$ Wasserstoff bedeuten, und pharmazeutisch annehmbare Salze davon.

**5.** Verbindungen gemäss Anspruch 1 der Formel I A, worin Z Sauerstoff oder Schwefel, $R_1$ und $R_2$ $C_1$-$C_4$-Alkyl, $R_3$ Hydroxy, $C_1$-$C_4$-Alkoxy, Benzyloxy, Acetoxy, Benzoyloxy, tert.-Butoxycarbonyloxy, Carbamoyloxy oder Mono- oder Di-$C_1$-$C_4$-alkylcarbamoyloxy, $R_4$ Wasserstoff, $C_1$-$C_4$-Alkyl oder Halogen in 7- oder 8-Stellung und $R_5$ Wasserstoff bedeuten, und pharmazeutisch annehmbare Salze davon.

**6.** Verbindungen gemäss Anspruch 1 der Formel

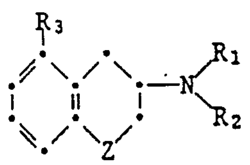

(I B),

worin Z Sauerstoff oder Schwefel, $R_1$ und $R_2$ $C_1$-$C_4$-Alkyl, Benzyl oder 2-Phenylethyl und $R_3$ Hydroxy, $C_1$-$C_4$-Alkoxy, Benzyloxy, Acetoxy, Benzoyloxy, tert.-Butoxycarbonyloxy, Carbamoyloxy oder Mono- oder Di-$C_1$-$C_4$-alkylcarbamoyloxy bedeuten, und pharmazeutisch annehmbare Salze davon.

**7.** Verbindungen gemäss Anspruch 6 der Formel I B, worin Z Sauerstoff bedeutet, und pharmazeutisch annehmbare Salze davon.

**8.** Verbindungen gemäss Anspruch 6 der Formel I B, worin Z Schwefel bedeutet, und pharmazeutisch annehmbare Salze davon.

**9.** Verbindungen gemäss Anspruch 6 der Formel I B, worin Z Sauerstoff oder Schwefel, $R_1$ und $R_2$ geradkettiges $C_1$-$C_4$-Alkyl mit identischen Bedeutungen und $R_3$ $C_1$-$C_4$-Alkoxy bedeuten, und pharmazeutisch annehmbare Salze davon.

**10.** 3,4-Dihydro-N,N-dipropyl-5-hydroxy-2H-[1]-benzothiopyran-3-amin und pharmazeutisch annehmbare Salze davon gemäss Anspruch 6.

**11.** 3,4-Dihydro-N,N-dipropyl-5-methoxy-2H-[1]-benzothiopyran-3-amin und pharmazeutisch annehmbare Salze davon gemäss Anspruch 6.

**12.** 3,4-Dihydro-N,N-dipropyl-5-äthoxy-2H-[1]-benzothiopyran-3-amin und pharmazeutisch annehmbare Salze davon gemäss Anspruch 6.

**13.** 3,4-Dihydro-N,N-dipropyl-5-propyloxy-2H-[1]-benzothiopyran-3-amin und pharmazeutisch annehmbare Salze davon gemäss Anspruch 6.

**14.** 3,4-Dihydro-N,N-dipropyl-5-hydroxy-2H-[1]-benzopyran-3-amin und pharmazeutisch annehmbare Salze

davon gemäss Anspruch 6.

**15.** 3,4-Dihydro-N,N-dipropyl-5-trimethylacetoxy-2H-[1]-benzopyran-3-amin und pharmazeutisch annehmbare Salze davon gemäss Anspruch 1.

**16.** 3,4-Dihydro-N,N-dipropyl-5-äthoxy-2H-[1]-benzopyran-3-amin und pharmazeutisch annehmbare Salze davon gemäss Anspruch 6.

**17.** Pharmazeutische Präparate enthaltend Verbindungen der Ansprüche 1-16 oder pharmazeutisch annehmbare Salze davon zusammen mit einem pharmazeutisch geeigneten Trägermaterial.

**18.** Die in den Ansprüchen 1-16 genannten Verbindungen zur Anwendung als Serotoninagonisten in einem Verfahren zur Behandlung des menschlichen oder tierischen Körpers von Depressionen, Wahrnehmungsstörungen und Kleinhirnstörungen und von Angstzuständen.

**19.** Verwendung der in den Ansprüchen 1 bis 16 genannten Verbindungen zur Herstellung von pharmazeutischen Präparaten für die therapeutische Behandlung von Depressionen, Wahrnehmungsstörungen und Kleinhirnstörungen.

**20.** Verfahren zur Herstellung der in Anspruch 1 genannten Verbindungen der Formel I A, in der die Symbole die in Anspruch 1 angegebenen Bedeutungen haben, und deren Salzen, dadurch gekennzeichnet, dass man

a) eine Verbindung der Formel

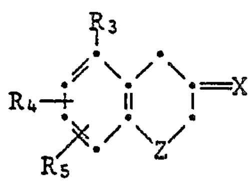

(II),

worin Z und $R_3$-$R_5$ die genannten Bedeutungen haben und X Oxo oder reaktionsfähiges, verestertes Hydroxy zusammen mit Wasserstoff, welches an die Stelle der Doppelbindung tritt, bedeuten, oder ein Mono- oder Di-S-Oxid einer Verbindung der Formel II, worin Z Schwefel bedeutet, mit einer Verbindung der Formel

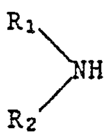

(III),

worin $R_1$ und $R_2$ die genannten Bedeutungen haben, kondensiert oder, falls X Oxo bedeutet, unter reduzierenden Bedingungen kondensiert, oder

b) eine Verbindung der Formel

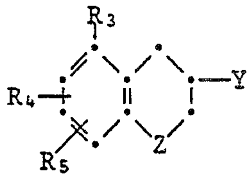

(IV),

worin Z und $R_3$-$R_5$ die genannten Bedeutungen haben und Y $-NH_2$, $-NHR_1$ oder $-NHR_2$ darstellt, mit einem reaktionsfähigen Ester eines Alkohols der Formel

$R_1$-OH oder $R_2$-OH (V),

worin jeweils $R_1$ und $R_2$ die genannten Bedeutungen haben, alkyliert oder mit einem dem Alkohol $R_1$-OH oder $R_2$-OH entsprechenden Aldehyd unter Einhaltung von reduzierenden Bedingungen alkyliert, oder

c) zur Herstellung einer Verbindung I A, worin $R_3$ Hydroxy bedeutet, eine Verbindung der Formel

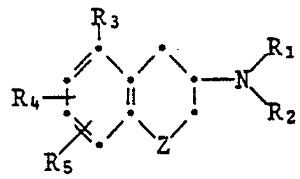

(VI),

worin Z, $R_1$, $R_2$, $R_4$ und $R_5$ die genannten Bedeutungen haben und $R_3$ veräthertes Hydroxy bedeutet, spaltet, oder

d) eine Verbindung der Formel

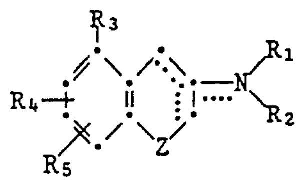

(VII),

worin Z und $R_1$-$R_5$ die genannten Bedeutungen haben und die punktierten Bindungslinien die mögliche Lage einer Doppelbindung in einer der angezeigten Positionen darstellen, reduziert, oder

e) eine Verbindung der Formel

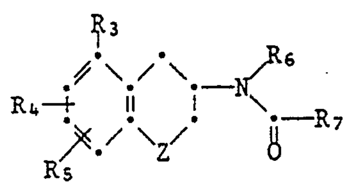

(VIII),

worin $R_6$ die Bedeutung von $R_1$ oder $R_2$ hat, Z und $R_1$-$R_5$ die genannten Bedeutungen haben und $R_7$ Wasserstoff, $C_1$-$C_3$-Alkyl, Phenyl oder Benzyl bedeuten, reduziert,

und, falls erwünscht, reaktionsfähige funktionelle Gruppen bei der Ausführung einer der genannten Verfahrensvarianten schützt und anschliessend die freie Verbindung der Formel I A isoliert und/oder eine erhältliche Verbindung der Formel I A in eine andere Verbindung der Formel I A, worin Z und $R_1$-$R_5$ die genannten Bedeutungen haben, umwandelt und/oder eine erhältliche freie Verbindung in ein Salz oder ein Salz in die freie Verbindung oder in ein anderes Salz umwandelt und/oder ein erhältliches Gemisch von Isomeren in einzelne Isomere auftrennt und/oder ein Racemat in die optischen Antipoden aufspaltet.

Patentansprüche für folgenden Vertragsstaat: AT

1. Verfahren zur Herstellung von Verbindungen der Formel

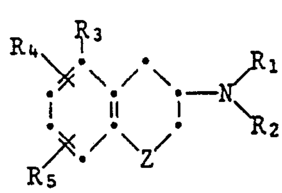

(I A),

worin Z Sauerstoff oder Schwefel, $R_1$ Wasserstoff, $C_1$-$C_4$-Alkyl, Benzyl oder 2-Phenylethyl, $R_2$ Wasserstoff, $C_1$-$C_4$-Alkyl, Benzyl oder 2-Phenylethyl oder $R_1$ und $R_2$ zusammengenommen Alkylen mit 4 bis 6 C-Atomen, $R_3$ Hydroxy, $C_1$-$C_4$-Alkoxy, Phenyl-$C_1$-$C_4$-alkoxy, welches am Phenylring durch 1-3 $C_1$-$C_4$-Alkyl, Halogen oder $C_1$-$C_4$-Alkoxy substituiert sein kann, $C_1$-$C_4$-Alkanoyloxy, Benzoyloxy, $C_1$-$C_4$-Alkoxycarbonyloxy, Carbamoyloxy oder Mono- oder Di-$C_1$-$C_4$-alkylcarbamoyloxy und $R_4$ und $R_5$ unabhängig voneinander Wasserstoff, $C_1$-$C_4$-Alkyl oder Halogen bedeuten, von Mono- oder Di-S-Oxiden dieser Verbindungen, worin Z Schwefel bedeutet, und von pharmazeutisch annehmbaren Salzen dieser Verbindungen, dadurch gekennzeichnet, dass man

a) eine Verbindung der Formel

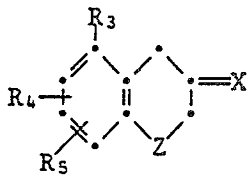

(II),

worin Z und $R_3$-$R_5$ die genannten Bedeutungen haben und X Oxo oder reaktionsfähiges, verestertes Hydroxy zusammen mit Wasserstoff, welches an die Stelle der Doppelbindung tritt, bedeuten, oder ein Mono- oder Di-S-Oxid einer Verbindung der Formel II, worin Z Schwefel bedeutet, mit einer Verbindung der Formel

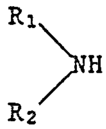

(III),

worin $R_1$ und $R_2$ die genannten Bedeutungen haben, kondensiert oder, falls X Oxo bedeutet, unter reduzierenden Bedingungen kondensiert, oder

b) eine Verbindung der Formel

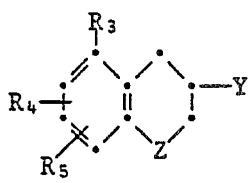

(IV),

worin Z und $R_3$-$R_5$ die genannten Bedeutungen haben und Y $-NH_2$, $-NHR_1$ oder $-NHR_2$ darstellt, mit einem reaktionsfähigen Ester eines Alkohols der Formel

$$R_1-OH \text{ oder } R_2-OH \qquad (V),$$

worin jeweils $R_1$ und $R_2$ die genannten Bedeutungen haben, alkyliert oder mit einem dem Alkohol $R_1$-OH oder $R_2$-OH entsprechenden Aldehyd unter Einhaltung von reduzierenden Bedingungen alkyliert, oder

c) zur Herstellung einer Verbindung I A, worin $R_3$ Hydroxy bedeutet, eine Verbindung der Formel

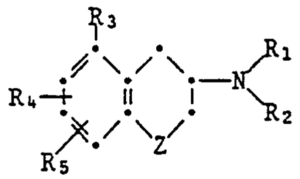

(VI),

worin Z, $R_1$, $R_2$, $R_4$ und $R_5$ die genannten Bedeutungen haben und $R_3$ veräthertes Hydroxy bedeutet, spaltet, oder
d) eine Verbindung der Formel

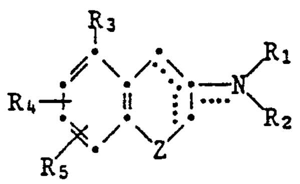

(VII),

worin Z und $R_1$-$R_5$ die genannten Bedeutungen haben und die punktierten Bindungslinien die mögliche Lage einer Doppelbindung in einer der angezeigten Positionen darstellen, reduziert, oder
e) eine Verbindung der Formel

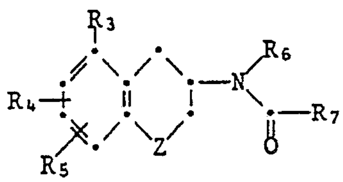

(VIII),

worin $R_6$ die Bedeutung von $R_1$ oder $R_2$ hat, Z und $R_1$-$R_5$ die genannten Bedeutungen haben und $R_7$ Wasserstoff, $C_1$-$C_3$-Alkyl, Phenyl oder Benzyl bedeuten, reduziert,
und, falls erwünscht, reaktionsfähige funktionelle Gruppen bei der Ausführung einer der genannten Verfahrensvarianten schützt und anschliessend die freie Verbindung der Formel I A isoliert und/oder eine erhältliche Verbindung der Formel I A in eine andere Verbindung der Formel I A, worin Z und $R_1$-$R_5$ die genannten Bedeutungen haben, umwandelt und/oder eine erhältliche freie Verbindung in ein Salz oder ein Salz in die freie Verbindung oder in ein anderes Salz umwandelt und/oder ein erhältliches Gemisch von Isomeren in einzelne Isomere auftrennt und/oder ein Racemat in die optischen Antipoden aufspaltet.

2. Verfahren gemäss Anspruch 1 zur Herstellung von Verbindungen der Formel I A, worin Z Sauerstoff bedeutet, und von pharmazeutisch annehmbaren Salzen davon.

3. Verfahren gemäss Anspruch 1 zur Herstellung von Verbindungen der Formel I A, worin Z Schwefel bedeutet, und von pharmazeutisch annehmbaren Salzen davon.

4. Verfahren gemäss Anspruch 1 zur Herstellung von Verbindungen der Formel I A, worin Z Sauerstoff oder Schwefel, $R_1$ Wasserstoff oder $C_1$-$C_4$-Alkyl, $R_2$ $C_1$-$C_4$-Alkyl, Benzyl oder 2-Phenylethyl oder $R_1$ und $R_2$ zusammen Butylen oder Pentylen, $R_3$ Hydroxy, $C_1$-$C_4$-Alkoxy, Benzyloxy, Acetoxy, Benzoyloxy, tert.-Butoxycarbonyloxy, Carbamoyloxy oder Mono- oder Di-$C_1$-$C_4$-alkylcarbamoyloxy, $R_4$ Wasserstoff, Halogen oder $C_1$-$C_4$-Alkyl und $R_5$ Wasserstoff bedeuten, und von pharmazeutisch annehmbaren Salzen davon.

5. Verfahren gemäss Anspruch 1 zur Herstellung von Verbindungen der Formel I A, worin Z Sauerstoff oder Schwefel, $R_1$ und $R_2$ $C_1$-$C_4$-Alkyl, $R_3$ Hydroxy, $C_1$-$C_4$-Alkoxy, Benzyloxy, Acetoxy, Benzoyloxy, tert.-Butoxycarbonyloxy, Carbamoyloxy oder Mono- oder Di-$C_1$-$C_4$-alkylcarbamoyloxy, $R_4$ Wasserstoff, $C_1$-$C_4$-Alkyl oder Halogen in 7- oder 8-Stellung und $R_5$ Wasserstoff bedeuten, und von pharmazeutisch

annehmbaren Salzen davon.

6. Verfahren gemäss Anspruch 1 zur Herstellung einer Verbindung der Formel

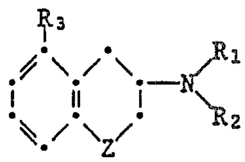

(I B),

worin Z Sauerstoff oder Schwefel, $R_1$ und $R_2$ $C_1$-$C_4$-Alkyl, Benzyl oder 2-Phenylethyl und $R_3$ Hydroxy, $C_1$-$C_4$-Alkoxy, Benzyloxy, Acetoxy, Benzoyloxy, tert.-Butoxycarbonyloxy, Carbamoyloxy oder Mono- oder Di-$C_1$-$C_4$-alkylcarbamoyloxy bedeuten, und von pharmazeutisch annehmbaren Salzen davon.

7. Verfahren gemäss Anspruch 6 zur Herstellung von Verbindungen der Formel I B, worin Z Sauerstoff bedeutet, und von pharmazeutisch annehmbaren Salzen davon.

8. Verfahren gemäss Anspruch 6 zur Herstellung von Verbindungen der Formel I B, worin Z Schwefel bedeutet, und von pharmazeutisch annehmbaren Salzen davon.

9. Verfahren gemäss Anspruch 6 zur Herstellung von Verbindungen der Formel I B, worin Z Sauerstoff oder Schwefel, $R_1$ und $R_2$ geradkettiges $C_1$-$C_4$-Alkyl mit identischen Bedeutungen und $R_3$ $C_1$-$C_4$-Alkoxy bedeuten, und von pharmazeutisch annehmbaren Salzen davon.

10. Verfahren gemäss Anspruch 6 zur Herstellung von 3,4-Dihydro-N,N-dipropyl-5-hydroxy-2H-[1]-benzothiopyran-3-amin, und von pharmazeutisch annehmbaren Salzen davon.

11. Verfahren gemäss Anspruch 6 zur Herstellung von 3,4-Dihydro-N,N-dipropyl-5-methoxy-2H-[1]-benzothiopyran-3-amin, und von pharmazeutisch annehmbaren Salzen davon.

12. Verfahren gemäss Anspruch 6 zur Herstellung von 3,4-Dihydro-N,N-dipropyl-5-äthoxy-2H-[1]-benzothiopyran-3-amin, und von pharmazeutisch annehmbaren Salzen davon.

13. Verfahren gemäss Anspruch 6 zur Herstellung von 3,4-Dihydro-N,N-dipropyl-5-propyloxy-2H-[1]-benzothiopyran-3-amin, und von pharmazeutisch annehmbaren Salzen davon.

14. Verfahren gemäss Anspruch 6 zur Herstellung von 3,4-Dihydro-N,N-dipropyl-5-hydroxy-2H-[1]-benzopyran-3-amin, und von pharmazeutisch annehmbaren Salzen davon.

15. Verfahren gemäss Anspruch 1 zur Herstellung von 3,4-Dihydro-N,N-dipropyl-5-trimethylacetoxy-2H-[1]-benzopyran-3-amin, und von pharmazeutisch annehmbaren Salzen davon.

16. Verfahren gemäss Anspruch 6 zur Herstellung von 3,4-Dihydro-N,N-dipropyl-5-äthoxy-2H-[1]-benzopyran-3-amin, und von pharmazeutisch annehmbaren Salzen davon.

17. Verfahren zur Herstellung von pharmazeutischen Präparaten, dadurch gekennzeichnet, dass man gemäss einem der Ansprüche 1 bis 16 hergestellte Verbindungen oder deren pharmazeutisch annehmbare Salze mit einem geeigneten Träger mischt.

**Claims**

1. A compound of the formula

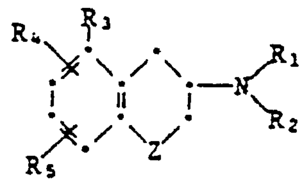

(I A),

wherein Z represents oxygen or sulfur, $R_1$ represents hydrogen, $C_1$-$C_4$alkyl, benzyl or 2-phenethyl, $R_2$ represents hydrogen, $C_1$-$C_4$alkyl, benzyl or 2-phenethyl, or $R_1$ and $R_2$ together represent alkylene having from 4 to 6 carbon atoms, $R_3$ represents hydroxy, $C_1$-$C_4$alkoxy, phenyl-$C_1$-$C_4$alkoxy which may be substituted at the phenyl ring by 1-3 $C_1$-$C_4$alkyl, halogen or $C_1$-$C_4$alkoxy, $C_1$-$C_4$alkanoyloxy, benzoyloxy, $C_1$-$C_4$alkoxycarbonyloxy, carbamoyloxy or mono- or di-$C_1$-$C_4$alkylcarbamoyloxy, and $R_4$ and $R_5$ each independently represents hydrogen, $C_1$-$C_4$alkyl or halogen, a mono- or di-S-oxide of such a compound wherein Z represents sulfur, or a pharmaceutically acceptable salt thereof.

**2.** A compound according to claim 1 of formula I A, wherein Z represents oxygen, or a pharmaceutically acceptable salt thereof.

**3.** A compound according to claim 1 of formula I A, wherein Z represents sulfur, or a pharmaceutically acceptable salt thereof.

**4.** A compound according to claim 1 of formula I A, wherein Z represents oxygen or sulfur, $R_1$ represents hydrogen or $C_1$-$C_4$alkyl, $R_2$ represents $C_1$-$C_4$alkyl, benzyl or 2-phenethyl, or $R_1$ and $R_2$ together represent butylene or pentylene, $R_3$ represents hydroxy, $C_1$-$C_4$alkoxy, benzyloxy, acetoxy, benzoyloxy, tert-butoxycarbonyloxy, carbamoyloxy or mono- or di-$C_1$-$C_4$alkylcarbamoyloxy, $R_4$ represents hydrogen, halogen or $C_1$-$C_4$alkyl and $R_5$ represents hydrogen, or a pharmaceutically acceptable salt thereof.

**5.** A compound according to claim 1 of formula I A, wherein Z represents oxygen or sulfur, $R_1$ and $R_2$ represent $C_1$-$C_4$alkyl, $R_3$ represents hydroxy, $C_1$-$C_4$alkoxy, benzyloxy, acetoxy, benzoyloxy, tert-butoxycarbonyloxy, carbamoyloxy or mono- or di-$C_1$-$C_4$alkylcarbamoyloxy, $R_4$ represents hydrogen, $C_1$-$C_4$alkyl or halogen in the 7- or 8-position, and $R_5$ represents hydrogen, or a pharmaceutically acceptable salt thereof.

**6.** A compound according to claim 1 of the formula

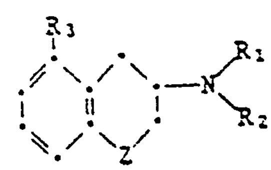

(IB)

wherein Z represents oxygen or sulfur, $R_1$ and $R_2$ represent $C_1$-$C_4$alkyl, benzyl or 2-phenethyl, and $R_3$ represents hydroxy, $C_1$-$C_4$alkoxy, benzyloxy, acetoxy, benzoyloxy, tert-butoxycarbonyloxy, carbamoyloxy or mono- or di-$C_1$-$C_4$alkylcarbamoyloxy, or a pharmaceutically acceptable salt thereof.

**7.** A compound according to claim 6 of the formula I B, wherein Z represents oxygen, or a pharmaceutically acceptable salt thereof.

**8.** A compound according to claim 6 of the formula I B, wherein Z represents sulfur, or a pharmaceutically acceptable salt thereof.

**9.** A compound according to claim 6 of the formula I B, wherein Z represents oxygen or sulfur, $R_1$ and $R_2$ are identical and represent straight-chain $C_1$-$C_4$alkyl, and $R_3$ represents $C_1$-$C_4$alkoxy, or a pharmaceutically acceptable salt thereof.

10. 3,4-Dihydro-N,N-dipropyl-5-hydroxy-2H-[1]-benzothiopyran-3-amine or a pharmaceutically acceptable salt thereof according to claim 6.

11. 3,4-Dihydro-N,N-dipropyl-5-methoxy-2H-[1]-benzothiopyran-3-amine or a pharmaceutically acceptable salt thereof according to claim 6.

12. 3-4-Dihydro-N,N-dipropyl-5-ethoxy-2H-[1]-benzothiopyran-3-amine or a pharmaceutically acceptable salt thereof according to claim 6.

13. 3,4-Dihydro-N,N-dipropyl-5-propyloxy-2H-[1]-benzothiopyran-3-amine or a pharmaceutically acceptable salt thereof according to claim 6.

14. 3,4-Dihydro-N,N-dipropyl-5-hydroxy-2H-[1]-benzopyran-3-amine or a pharmaceutically acceptable salt thereof according to claim 6.

15. 3,4-Dihydro-N,N-dipropyl-5-trimethylacetoxy-2H-[1]-benzopyran-3-amine or a pharmaceutically acceptable salt thereof according to claim 1.

16. 3,4-Dihydro-N,N-dipropyl-5-ethoxy-2H-[1]-benzopyran-3-amine or a pharmaceutically acceptable salt thereof according to claim 6.

17. A pharmaceutical preparation comprising a compound according to claims 1-16 or a pharmaceutically acceptable salt thereof together with a pharmaceutically acceptable carrier.

18. The compounds defined in claims 1-16 for use as serotonin agonists in a method for the treatment of the human or animal body for depression, perceptual disorders and cerebellar disorders, and anxiety states.

19. The use of the compounds defined in claims 1 to 16 for the manufacture of pharmaceutical preparations for the therapeutic treatment of depression, perceptual disorders and cerebellar disorders.

20. A process for the manufacture of a compound of formula I A defined in claim 1 in which the symbols are as defined in claim 1, or a salt thereof, which comprises:

a) condensing a compound of the formula

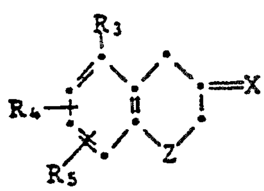

(II),

wherein Z and $R_3$ to $R_5$ are as defined and X represents oxo or reactive esterified hydroxy together with hydrogen, which takes the place of the double bond, or a mono- or di-S-oxide of a compound of formula II wherein Z represents sulfur, with a compound of the formula

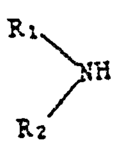

(III),

wherein $R_1$ and $R_2$ are as defined, the condensation being carried out under reducing conditions if X represents oxo, or

b) alkylating a compound of the formula

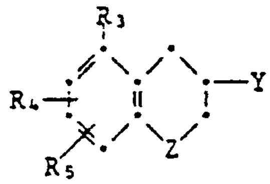

(IV),

wherein Z and $R_3$ to $R_5$ are as defined and Y represents $-NH_2$, $-NHR_1$ or $-NHR_2$, with a reactive ester of an alcohol of the formula

$$R_1-OH \text{ or } R_2-OH \qquad (V),$$

wherein $R_1$ and $R_2$ are as defined, or with an aldehyde corresponding to the alcohol $R_1$-OH or $R_2$-OH under reducing conditions, or

c) for the manufacture of a compound I A in which $R_3$ represents hydroxy, cleaving a compound of the formula

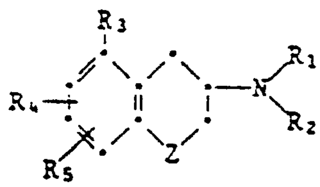

(VI),

wherein Z, $R_1$, $R_2$, $R_4$ and $R_5$ are as defined and $R_3$ represents etherified hydroxy, or

d) reducing a compound of the formula

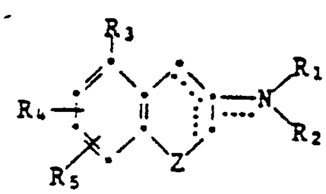

(VII),

wherein Z and $R_1$ to $R_5$ are as defined and the dotted bond lines represent the possible position of a double bond in one of the positions indicated, or

e) reducing a compound of the formula

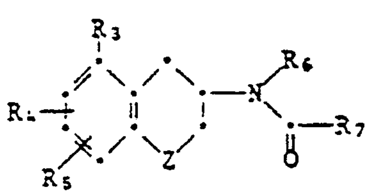

(VIII),

wherein $R_6$ represents $R_1$ or $R_2$, Z and $R_1$ to $R_5$ are as defined and $R_7$ represents hydrogen, $C_1$-$C_3$-alkyl, phenyl or benzyl,

and, if desired, protecting reactive functional groups when carrying out any of the mentioned process variants, and then isolating the free compound of formula I A, and/or converting an obtainable compound of formula I A into a different compound of formula I A in which Z and $R_1$ to $R_5$ are as defined, and/or converting an obtainable free compound into a salt or a salt into the free compound or into a different salt, and/or separating an obtainable mixture of isomers into individual isomers, and/or resolving a racemate into the optical antipodes.

Claims for the following Contracting State: AT

1. A process for the manufacture of a compound of the formula

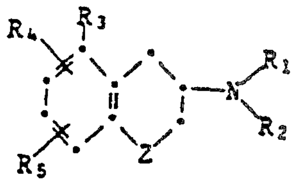

(I A),

wherein Z represents oxygen or sulfur, $R_1$ represents hydrogen, $C_1$-$C_4$alkyl, benzyl or 2-phenethyl, $R_2$ represents hydrogen, $C_1$-$C_4$alkyl, benzyl or 2-phenethyl, or $R_1$ and $R_2$ together represent alkylene having from 4 to 6 carbon atoms, $R_3$ represents hydroxy, $C_1$-$C_4$alkoxy, phenyl$C_1$-$C_4$alkoxy which may be substituted at the phenyl ring by 1-3 $C_1$-$C_4$alkyl, halogen or $C_1$-$C_4$alkoxy, $C_1$-$C_4$alkanoyloxy, benzoyloxy, $C_1$-$C_4$alkoxycarbonyloxy, carbamoyloxy or mono- or di-$C_1$-$C_4$alkylcarbamoyloxy, and $R_4$ and $R_5$ each independently represents hydrogen, $C_1$-$C_4$alkyl or halogen, a mono- or di-S-oxide of such a compound wherein Z represents sulfur, or a pharmaceutically acceptable salt thereof, which comprises:

a) condensing a compound of the formula

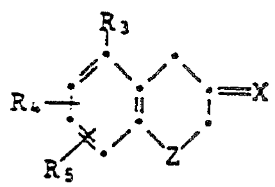

(II),

wherein Z and $R_3$ to $R_5$ are as defined and X represents oxo or reactive esterified hydroxy together with hydrogen, which takes the place of the double bond, or a mono- or di-S-oxide of a compound of formula II wherein Z represents sulfur, with a compound of the formula

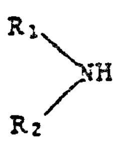

(III),

wherein $R_1$ and $R_2$ are as defined, the condensation being carried out under reducing conditions if X represents oxo, or

b) alkylating a compound of the formula

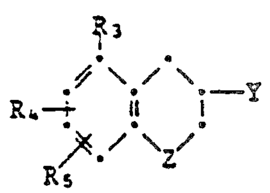

(IV),

wherein Z and $R_3$ to $R_5$ are as defined and Y represents $-NH_2$, $-NHR_1$ or $-NHR_2$, with a reactive ester of an alcohol of the formula

$$R_1-OH \text{ or } R_2-OH \qquad (V),$$

wherein $R_1$ and $R_2$ are as defined, or with an aldehyde corresponding to the alcohol $R_1$-OH or $R_2$-OH under reducing conditions, or

c) for the manufacture of a compound I A in which $R_3$ represents hydroxy, cleaving a compound of

the formula

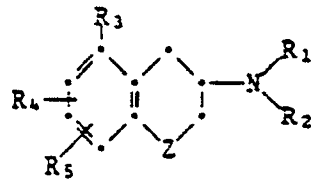

(VI),

wherein Z, $R_1$, $R_2$, $R_4$ and $R_5$ are as defined and $R_3$ represents etherified hydroxy, or
d) reducing a compound of the formula

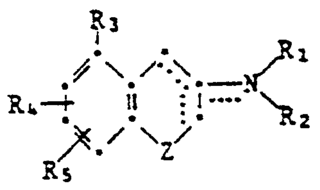

(VII),

wherein Z and $R_1$ to $R_5$ are as defined and the dotted bond lines represent the possible position of a double bond in one of the positions indicated, or
e) reducing a compound of the formula

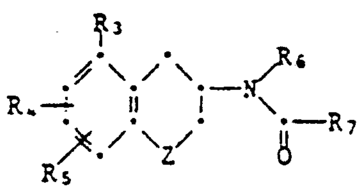

(VIII),

wherein $R_6$ represents $R_1$ or $R_2$, Z and $R_1$ to $R_5$ are as defined and $R_7$ represents hydrogen, $C_1$-$C_3$alkyl, phenyl or benzyl,
and, if desired, protecting reactive functional groups when carrying out any of the mentioned process variants, and then isolating the free compound of formula I A, and/or converting an obtainable compound of formula I A into a different compound of formula I A in which Z and $R_1$ to $R_5$ are as defined, and/or converting an obtainable free compound into a salt or a salt into the free compound or into a different salt, and/or separating an obtainable mixture of isomers into individual isomers, and/or resolving a racemate into the optical antipodes.

**2.** A process according to claim 1 for the manufacture of a compound of formula I A, wherein Z represents oxygen, or a pharmaceutically acceptable salt thereof.

**3.** A process according to claim 1 for the manufacture of a compound of formula I A, wherein Z represents sulfur, or a pharmaceutically acceptable salt thereof.

**4.** A process according to claim 1 for the manufacture of a compound of formula I A, wherein Z represents oxygen or sulfur, $R_1$ represents hydrogen or $C_1$-$C_4$alkyl, $R_2$ represents $C_1$-$C_4$alkyl, benzyl or 2-phenethyl, or $R_1$ and $R_2$ together represent butylene or pentylene, $R_3$ represents hydroxy, $C_1$-$C_4$alkoxy, benzyloxy, acetoxy, benzoyloxy, tert-butoxycarbonyloxy, carbamoyloxy or mono- or di-$C_1$-$C_4$alkylcarbamoyloxy, $R_4$ represents hydrogen, halogen or $C_1$-$C_4$alkyl and $R_5$ represents hydrogen, or a pharmaceutically acceptable salt thereof.

**5.** A process according to claim 1 for the manufacture of a compound of formula I A, wherein Z represents oxygen or sulfur, $R_1$ and $R_2$ represent $C_1$-$C_4$alkyl, $R_3$ represents hydroxy, $C_1$-$C_4$alkoxy, benzyloxy, acetoxy, benzoyloxy, tert-butoxycarbonyloxy, carbamoyloxy or mono- or di-$C_1$-$C_4$alkylcarbamoyloxy, $R_4$ represents hydrogen, $C_1$-$C_4$alkyl or halogen in the 7- or 8-position, and $R_5$ represents hydrogen, or a pharmaceutically acceptable salt thereof.

6. A process according to claim 1 for the manufacture of a compound of the formula

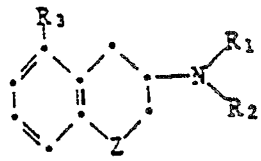

(IB)

wherein Z represents oxygen or sulfur, $R_1$ and $R_2$ represent $C_1$-$C_4$alkyl, benzyl or 2-phenethyl, and $R_3$ represents hydroxy, $C_1$-$C_4$alkoxy, benzyloxy, acetoxy, benzoyloxy, tert-butoxycarbonyloxy, carbamoyloxy or mono- or di$C_1$-$C_4$alkylcarbamoyloxy, or a pharmaceutically acceptable salt thereof.

7. A process according to claim 6 for the manufacture of a compound of the formula I B, wherein Z represents oxygen, or a pharmaceutically acceptable salt thereof.

8. A process according to claim 6 for the manufacture of a compound of the formula I B, wherein Z represents sulfur, or a pharmaceutically acceptable salt thereof.

9. A process according to claim 6 for the manufacture of a compound of the formula I B, wherein Z represents oxygen or sulfur, $R_1$ and $R_2$ are identical and represent straight-chain $C_1$-$C_4$alkyl, and $R_3$ represents $C_1$-$C_4$alkoxy, or a pharmaceutically acceptable salt thereof.

10. A process according to claim 6 for the manufacture of 3,4-dihydro-N,N-dipropyl-5-hydroxy-2H-[1]-benzothiopyran-3-amine or a pharmaceutically acceptable salt thereof.

11. A process according to claim 6 for the manufacture of 3,4-dihydro-N,N-dipropyl-5-methoxy-2H-[1]-benzothiopyran-3-amine or a pharmaceutically acceptable salt thereof.

12. A process according to claim 6 for the manufacture of 3,4-dihydro-N,N-dipropyl-5-ethoxy-2H-[1]-benzothiopyran-3-amine or a pharmaceutically acceptable salt thereof.

13. A process according to claim 6 for the manufacture of 3,4-dihydro-N,N-dipropyl-5-propyloxy-2H-[1]-benzothiopyran-3-amine or a pharmaceutically acceptable salt thereof.

14. A process according to claim 6 for the manufacture of 3,4-dihydro-N,N-dipropyl-5-hydroxy-2H-[1]-benzopyran-3-amine or a pharmaceutically acceptable salt thereof.

15. A process according to claim 1 for the manufacture of 3,4-dihydro-N,N-dipropyl-5-trimethylacetoxy-2H-[1]-benzopyran-3-amine or a pharmaceutically acceptable salt thereof.

16. A process according to claim 6 for the manufacture of 3,4-dihydro-N,N-dipropyl-5-ethoxy-2H-[1]-benzopyran-3-amine or a pharmaceutically acceptable salt thereof.

17. A process for the manufacture of a pharmaceutical preparation, characterised in that a compound prepared in accordance with any one of claims 1 to 16 or a pharmaceutically acceptable salt thereof is mixed with a suitable carrier.

## Revendications

1. Composés de formule

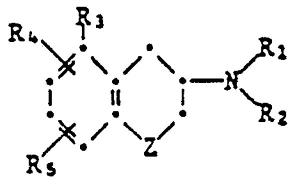

(I A),

où Z représente l'oxygène ou le soufre, $R_1$ représente l'hydrogène, un alkyle en $C_1$-$C_4$, le benzyle ou le 2-phényléthyle, $R_2$ représente l'hydrogène, un alkyle en $C_1$-$C_4$, le benzyle ou le 2-phényléthyle ou $R_1$ et $R_2$ pris ensemble représentent un alkylène ayant 4 à 6 atomes de C, $R_3$ représente l'hydroxy, un alcoxy en $C_1$-$C_4$, un phénylalcoxy en $C_1$-$C_4$ pouvant être substitué sur le noyau phényle par 1 à 3 alkyles en $C_1$-$C_4$, un halogène ou un alcoxy en $C_1$-$C_4$, un alcanoyle en $C_1$-$C_4$ oxy, un benzoyloxy, un alcoxy en $C_1$-$C_4$ carbonyloxy, un carbamoyloxy ou un mono- ou dialkyle en $C_1$-$C_4$ carbamoyloxy et $R_4$ et $R_5$ représentent indépendamment l'un de l'autre l'hydrogène, un alkyle en $C_1$-$C_4$ ou un haloène, un mono- ou un di-S-oxyde de ces composés ou Z représente le soufre et les sels pharmaceutiquement acceptables de ces composés.

2. Composés selon la revendication 1 de formule I A où Z représente l'oxygène et leurs sels pharmaceutiquement acceptables.

3. Composés selon la revendication 1 de formule I A où Z représente le soufre et leurs sels pharmaceutiquement acceptables.

4. Composés selon la revendication 1 de formule I A où Z représente l'oxygène ou le soufre, $R_1$ représente l'hydrogène ou un alkyle en $C_1$-$C_4$, $R_2$ représente un alkyle en $C_1$-$C_4$, le benzyle, ou le 2-phényléthyle ou $R_1$ et $R_2$ représentent ensemble le butylène ou le pentylène, $R_3$ représente l'hydroxy, un alcoxy en $C_1$-$C_4$, le benzyloxy, l'acétoxy, le benzoyloxy, le tert.-butoxycarbonyloxy, un carbamoyloxy ou un mono- ou dialkyle en $C_1$-$C_4$ carbamoyloxy, $R_4$ représente l'hydrogène, un halogène ou un alkyle en $c_1$-$C_4$ et $R_5$ représente l'hydrogène et leurs sels pharmaceutiquement acceptables.

5. Composés selon la revendication 1 de formule I A où Z représente l'oxygène ou le soufre, $R_1$ et $R_2$ représentent un alkyle en $C_1$-$C_4$, $R_3$ représente l'hydroxy, un alcoxy en $C_1$-$C_4$, le benzyloxy, l'acétoxy, le benzoyloxy, le tert.-butoxycarbonyloxy, le carbamoyloxy ou un mono- ou dialkyle en $C_1$-$C_4$ carbamoyloxy, $R_4$ représente l'hydrogène, un alkyle en $C_1$-$C_4$ ou un halogène en position 7 ou 8 et $R_5$ représente l'hydrogène et leurs sels pharmaceutiquement acceptables.

6. Composés selon la revendication 1 de formule

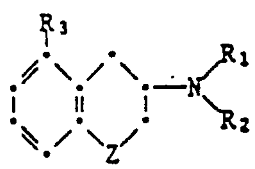

(I B),

où Z représente l'oxygène ou le soufre, $R_1$ et $R_2$ représentent un alkyle en $C_1$-$C_4$, le benzyle ou le 2-phényléthyle et $R_3$ représente l'hydroxy, un alcoxy en $C_1$-$C_4$, le benzyloxy, l'acétoxy, le benzoyloxy, le tert.-butoxycarbonyloxy, le carbamoyloxy ou un mono- ou dialkyle en $C_1$-$C_4$ carbamoyloxy et leurs sels pharmaceutiquement acceptables.

7. Composés selon la revendication 6 de formule I B où Z représente l'oxygène et leurs sels pharmaceutiquement acceptables.

8. Composés selon la revendication 6 de formule I B où Z représente le soufre et leurs sels pharmaceutiquement acceptables.

9. Composés selon la revendication 6 de formule I B où Z représente l'oxygène ou le soufre, $R_1$ et $R_2$-----

représentent un alkyle en $C_1$-$C_4$ à chaîne droite et ont la même signification et $R_3$ représente un alcoxy en $C_1$-$C_4$ et leurs sels pharmaceutiquement acceptables.

**10.** La 3,4-dihydro-N,N-dipropyl-5-hydroxy-2H-[1]-benzothiopyranne-3-amine et ses sels pharmaceutiquement acceptables selon la revendication 6.

**11.** La 3,4-dihydro-N-N-dipropyl-5-méthoxy-2H-[1]-benzothiopyranne-3-amine et ses sels pharmaceutiquement acceptables selon la revendication 6.

**12.** La 3-4-dihydro-N,N-dipropyl-5-éthoxy-2H-[1]-benzothiopyranne-3-amine et ses sels pharmaceutiquement acceptables selon la revendication 6.

**13.** La 3,4-dihydro-N,N-dipropyl-5-propyloxy-2H-[1]-benzothiopyranne-3-amine et ses sels pharmaceutiquement acceptables selon la revendication 6.

**14.** La 3,4-dihydro-N,N-dipropyl-5-hydroxy-2H-[1]-benzopyranne-3-amine et ses sels pharmaceutiquement acceptables selon la revendication 6.

**15.** La 3,4-dihydro-N,N-dipropyl-5-triméthylacétoxy-2H-[1]-benzopyranne-3-amine et ses sels pharmaceutiquement acceptables selon la revendication 6.

**16.** La 3,4-dihydro-N,N-dipropyl-5-éthoxy-2H-[1]-benzopyranne-3-amine et ses sels pharmaceutiquement acceptables selon la revendication 6.

**17.** Préparations pharmaceutiques contenant les composés des revendications 1 à 16 ou leurs sels pharmaceutiquement acceptables en association avec un matériau de support pharmaceutiquement approprié.

**18.** Composés définis dans les revendications 1 à 16 destinés à être utilisés comme agonistes de la sérotonine dans un procédé pour le traitement du corps humain ou animal des dépressions, troubles de la perception, troubles cérébillaires et états d'anxiété.

**19.** Utilisation des composés définis dans les revendications 1 à 16 pour l'obtention de préparations pharmaceutiques pour le traitement thérapeutique des dépressions, troubles de la perception et troubles cérébillaires.

**20.** Procédé pour la préparation des composés définis dans la revendication 1 de formule I A dans laquelle les symboles ont la signification indiquée dans la revendication 1, caractérisé

a) en ce que l'on condense un composé de formule

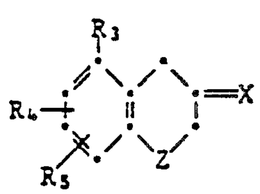

(II),

où Z et $R_3$-$R_5$ ont les significations précitées et X représente l'oxo ou un hydroxy estérifié réactif ensemble avec l'hydrogène qui se substitue à la liaison double ou un mono- ou di-S-oxyde d'un composé de formule II où Z représente le soufre avec un composé de formule

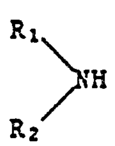

(III),

où $R_1$ et $R_2$ ont les significations précitées, la condensation s'effectuant dans des conditions réductrices, lorsque X représente l'oxo ou
b) en ce que l'on alcoyle un composé de formule

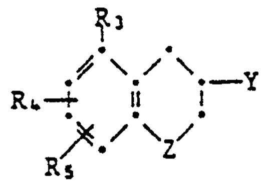

(IV),

——— où Z et $R_3$-$R_5$ ont les significations précitées et Y-représente $-NH_2$, $-NHR_1$ ou $-NHR_2$, avec un ester réactif d'un alcool de formule

$R_1-OH$ ou $R_2-OH$ (V)

où $R_1$ et $R_2$ ont les significations précitées ou avec un aldéhyde correspondant à l'alcool $R_1$-OH ou $R_2$-OH, en respectant des conditions réductrices ou
c) en ce que l'on clive, pour la préparation d'un composé I A où $R_3$ représente l'hydroxy, le composé de formule

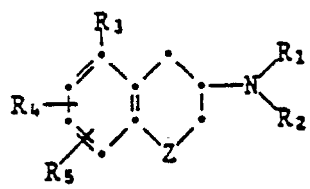

(VI),

où Z, $R_1$, $R_2$, $R_4$ et $R_5$ ont les significations précitées et $R_3$ représente un hydroxy éthéré ou
d) en ce que l'on réduit un composé de formule

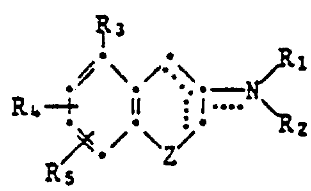

(VII),

où Z et $R_1$-$R_5$ ont les significations précitées et les liaisons en pointillés représentent la position possible d'une liaison double dans l'une des positions indiquées ou
e) en ce que l'on réduit un composé de formule

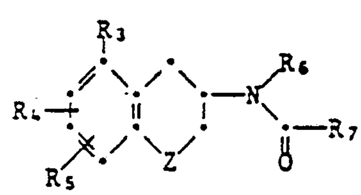

(VIII),

où $R_6$ a la signification de $R_1$ ou de $R_2$, Z et $R_1$-$R_5$ ont les significations précitées et $R_7$ représente l'hydrogène, un alkyle en $C_1$-$C_3$, le phényle ou le benzyle,
et, si désiré, en ce que l'on protège les groupes fonctionnels réactifs, lors de l'exécution de l'une des variantes du procédé précitées et en ce que l'on isole ensuite le composé libre de formule I A et/ou en ce que l'on transforme le composé de formule I A obtenu en un autre composé de formule I A où Z et $R_1$-$R_5$ ont les significations précitées et/ou en ce que l'on transforme le composé libre

obtenu en un sel ou un sel en un composé libre ou en un autre sel et/ou en ce que l'on sépare le mélange d'isomères obtenu en isomères individuels et/ou en ce que l'on sépare un racémate en ses antipodes optiques.

Revendications pour l'Etat contractant suivant: AT

1. Procédé pour la préparation des composés de formule

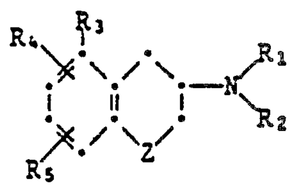

(I A),

où Z représente l'oxygène ou le soufre, $R_1$ représente l'hydrogène, un alkyle en $C_1$-$C_4$, le benzyle ou le 2-phényléthyle, $R_2$ représente l'hydrogène, un alkyle en $C_1$-$C_4$, le benzyle ou le 2-phényléthyle ou $R_1$ et $R_2$ pris ensemble représentent un alkylène ayant 4 à 6 atomes de C, $R_3$ représente l'hydroxy, un alcoxy en $C_1$-$C_4$, un phénylalcoxy en $C_1$-$C_4$ pouvant être substitué sur le noyau phényle par 1 à 3 alkyles en $C_1$-$C_4$, un halogène ou un alcoxy en $C_1$-$C_4$, un alcanoyle en $C_1$-$C_4$ oxy, un benzoyloxy, un alcoxy en $C_1$-$C_4$ carbonyloxy, un carbamoyloxy ou un mono- ou dialkyle en $C_1$-$C_4$ carbamoyloxy et $R_4$ et $R_5$ représentent indépendamment l'un de l'autre l'hydrogène, un alkyle en $C_1$-$C_4$ ou un halogène, un mono- ou un di-S-oxyde de ces composés ou Z représente le soufre et des sels pharmaceutiquement acceptables de ces composés, caractérisé

a) en ce que l'on condense un composé de formule

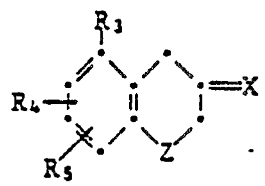

(II),

où Z et $R_3$-$R_5$ ont les significations précitées et X représente l'oxo ou un hydroxy estérifié réactif ensemble avec l'hydrogène qui se substitue à la liaison double ou un mono- ou di- S-oxyde d'un composé de formule II où Z représente le soufre avec un composé de formule

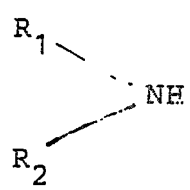

(III)

où $R_1$ et $R_2$ ont les significations précitées, la condensation s'effectuant dans des conditions réductrices, lorsque X représente l'oxo ou

b) en ce que l'on alcoyle un composé de formule

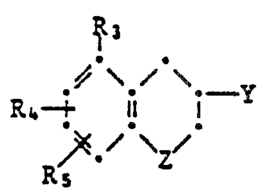

(IV),

où Z et $R_3$-$R_5$ ont les significations précitées et Y représente $-NH_2$, $-NHR_1$ ou $-NHR_2$, avec un ester réactif d'un alcool de formule

$$R_1\text{-OH} \quad \text{ou} \quad R_2\text{-OH} \qquad (V)$$

où $R_1$ et $R_2$ ont les significations précitées ou avec un aldéhyde correspondant à l'alcool $R_1$-OH ou $R_2$-OH, en respectant des conditions réductrices ou
c) en ce que l'on clive, pour la préparation d'un composé I A où $R_3$ représente l'hydroxy, le composé de formule

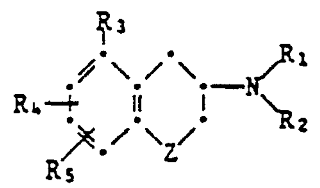

(VI),

où Z, $R_1$, $R_2$, $R_4$ et $R_5$ ont les significations précitées et $R_3$ représente un hydroxy éthéré ou
d) en ce que l'on réduit un composé de formule

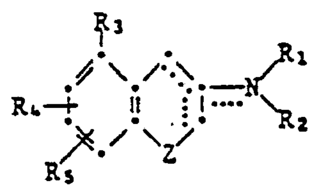

(VII),

où Z et $R_1$-$R_5$ ont les significations précitées et les liaisons en pointillés représentent la position possible d'une liaison double dans l'une des positions indiquées ou
e) en ce que l'on réduit un composé de formule

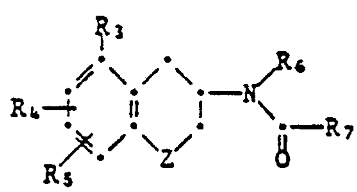

(VIII),

où $R_6$ a la signification de $R_1$ ou de $R_2$, Z et $R_1$-$R_5$ ont les significations précitées et $R_7$ représente l'hydrogène, un alkyle en $C_1$-$C_3$, le phényle ou le benzyle, et, si désiré, en ce que l'on protège les groupes fonctionnels réactifs, lors de l'exécution de l'une des variantes du procédé précitées et en ce que l'on isole ensuite le composé libre de formule I A et/ou en ce que l'on transforme le composé de formule I A obtenu en un autre composé de formule I A où Z et $R_1$-$R_5$ ont les significations précitées et/ou en ce que l'on transforme le composé libre obtenu en un sel ou un sel en un composé libre ou en un autre sel et/ou en ce que l'on sépare le mélange d'isomères obtenu en isomères individuels et/ou en ce que l'on sépare un racémate en ses antipodes optiques.

2. Procédé selon la revendication 1 pour la préparation des composés de formule I A où Z représente l'oxygène et de leurs sels pharmaceutiquement acceptables.

3. Procédé selon la revendication 1 pour la préparation des composés de formule I A où Z représente le soufre et de leurs sels pharmaceutiquement acceptables.

**4.** Procédé selon la revendication 1 pour la préparation des composés de formule I A où Z représente l'oxygène ou le soufre, $R_1$ représente l'hydrogène ou un alkyle en $C_1$-$C_4$, $R_2$ représente un alkyle en $C_1$-$C_4$, le benzyle, ou le 2-phényléthyle ou $R_1$ et $R_2$ représentent ensemble le butylène ou le pentylène, $R_3$ représente l'hydroxy, un alcoxy en $C_1$-$C_4$, le benzyloxy, l'acétoxy, le benzoyloxy, le tert.-butoxycarbonyloxy, un carbamoyloxy ou un mono- ou dialkyle en $C_1$-$C_4$ carbamoyloxy, $R_4$ représente l'hydrogène, un halogène ou un alkyle en $C_1$-$C_4$ et $R_5$ représente l'hydrogène et de leurs sels pharmaceutiquement acceptables.

**5.** Procédé selon la revendication 1 pour la préparation des composés de formule I A où Z représente l'oxygène ou le soufre, $R_1$ et $R_2$ représentent un alkyle en $C_1$-$C_4$, $R_3$ représente l'hydroxy, un alcoxy en $C_1$-$C_4$, le benzyloxy, l'acétoxy, le benzoyloxy, le tert.-butoxycarbonyloxy, le carbamoyloxy ou un mono- ou dialkyle en $C_1$-$C_4$ carbamoyloxy, $R_4$ représente l'hydrogène, un alkyle en $C_1$-$C_4$ ou un halogène en position 7 ou 8 et $R_5$ représente l'hydrogène et de leurs sels pharmaceutiquement acceptables.

**6.** Procédé selon la revendication 1 pour la préparation d'un composé de formule

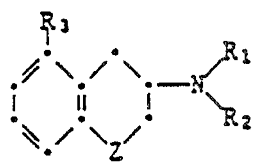

(I B),

où Z représente l'oxygène ou le soufre, $R_1$ et $R_2$ représentent un alkyle en $C_1$-$C_4$, le benzyle ou le 2-phényléthyle et $R_3$ représente l'hydroxy, un alcoxy en $C_1$-$C_4$, le benzyloxy, l'acétoxy, le benzoyloxy, le tert.-butoxycarbonyloxy, le carbamoyloxy ou un mono- ou dialkyle en $C_1$-$C_4$ carbamoyloxy et de leurs sels pharmaceutiquement acceptables.

**7.** Procédé selon la revendication 6 pour la préparation des composés de formule I B où Z représente l'oxygène et de leurs sels pharmaceutiquement acceptables.

**8.** Procédé selon la revendication 6 pour la préparation des composés de formule I B où Z représente le soufre et de leurs sels pharmaceutiquement acceptables.

**9.** Procédé selon la revendication 6 pour la préparation des composés de formule I B où Z représente l'oxygène ou le soufre, $R_1$ et $R_2$ représentent un alkyle en $C_1$-$C_4$ à chaîne droite et ont la même signification et $R_3$ représente un alcoxy en $C_1$-$C_4$ et de leurs sels pharmaceutiquement acceptables.

**10.** Procédé selon la revendication 6 pour la préparation de la 3,4-dihydro-N,N-dipropyl-5-hydroxy-2H-[1]-benzothiopyranne-3-amine et de ses sels pharmaceutiquement acceptables.

**11.** Procédé selon la revendication 6 pour la préparation de la 3,4-dihydro-N,N-dipropyl-5-méthoxy-2H-[1]-benzothiopyranne-3-amine et de ses sels pharmaceutiquement acceptables.

**12.** Procédé selon la revendication 6 pour la préparation de la 3,4-dihydro-N,N-dipropyl-5-éthoxy-2H-[1]-benzothiopyranne-3-amine et de ses sels pharmaceutiquement acceptables.

**13.** Procédé selon la revendication 6 pour la préparation de la 3,4-dihydro-N,N-dipropyl-5-propyloxy-2H-[1]-benzothiopyranne-3-amine et de ses sels pharmaceutiquementacceptables.

**14.** Procédé selon la revendication 6 pour la préparation de la 3,4-dihydro-N,N-dipropyl-5-hydroxy-2H-[1]-benzopyranne-3-amine et de ses sels pharmaceutiquement acceptables.

**15.** Procédé selon la revendication 1 pour la préparation de la 3,4-dihydro-N,N-dipropyl-5-triméthylacétoxy-2H-[1]-benzopyranne-3-amine et de ses sels pharmaceutiquement acceptables.

**16.** Procédé selon la revendication 6 pour la préparation de la 3,4-dipropyl-5-éthoxy-2H-[1]-benzopyranne-3-amine et de ses sels pharmaceutiquement acceptables.

**17.** Procédé pour l'obtention de préparations pharmaceutiques, caractérisé en ce que l'on mélange les composés préparés selon l'une des revendications 1 à 16 ou leurs sels pharmaceutiquement acceptables avec un support approprié.